(19) Europäisches Patentamt
European Patent Office
Office européen des brevets

(11) EP 2 359 818 A1

# (12) EUROPEAN PATENT APPLICATION

(43) Date of publication:
**24.08.2011 Bulletin 2011/34**

(21) Application number: **11157791.2**

(22) Date of filing: **13.02.2008**

(51) Int Cl.:
***A61K 31/00*** *(2006.01)* ***A61K 31/4045*** *(2006.01)*
***A61P 35/00*** *(2006.01)* ***A61K 31/5377*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.02.2007 US 890005 P**

(62) Document number(s) of the earlier application(s) in accordance with Art. 76 EPC:
**08729719.8 / 2 120 900**

(71) Applicant: **Novartis AG**
**4056 Basel (CH)**

(72) Inventors:
- **Atadja, Peter Wisdom**
  **Acton, MA 01720 (US)**
- **Shao, Wenlin**
  **Dedham, MA 02026 (US)**
- **Bhalla, Kapil N.**
  **Martinez, GA 30907 (US)**

(74) Representative: **Gruber, Markus**
**Novartis Pharma AG**
**Patent Department**
**4002 Basel (CH)**

Remarks:
This application was filed on 11-03-2011 as a divisional application to the application mentioned under INID code 62.

## (54) Combination of LBH589 with HSP 90 inhibitors for treating cancer

(57) The invention relates to a combination comprising the N-hydroxy-3-[4-[[[2-(2-methyl-1 H-indol-3-yl)-ethyl]-amino]methyl]phenyl]-2E-2-propenamide; and one or more pharmaceutically active agents; pharmaceutical compositions comprising said combination; methods of treatment comprising said combination; processes for making said combination; and a commercial package comprising said combination.

EP 2 359 818 A1

## Description

The invention relates to a combination comprising N-hydroxy-3-[4-[[[2-(2-methyl-1H-indol-3-yl)-ethyl]-amino] methyl]phenyl]-2E-2-propenamide; and one or more pharmaceutically active agents; pharmaceutical compositions comprising said combination; methods of treatment comprising said combination; processes for making said combination; and a commercial package comprising said combination.

## Background of the Invention

Reversible acetylation of histones is a major regulator of gene expression that acts by altering accessibility of transcription factors to DNA. In normal cells, histone deacetylase (HDA) and histone acetyltrasferase together control the level of acetylation of histones to maintain a balance. Inhibition of HDA results in the accumulation of hyperacetylated histones, which results in a variety of cellular responses. Inhibitors of HDA (HDAI) have been studied for their therapeutic effects on cancer cells. Recent developments in the field of HDAI research have provided active compounds, both highly efficacious and stable, that are suitable for treating tumors.
Accruing evidence suggests that HDAI are even more efficacious when used in combination with other chemotherapeutic agents. There are both synergistic and additive advantages, both for efficacy and safety. Therapeutic effects of combinations of chemotherapeutic agents with HDAI can result in lower safe dosages ranges of each component in the combination.

## Summary of the Invention

The invention relates to combination which comprises:

(a) N-hydroxy-3-[4-[[[2-(2-methyl-1 H-indol-3-yl)-ethyl]-amino]methyl]phenyl]-2E-2-propenamide; and

(b) one or more pharmaceutically active agents.

The invention further relates to pharmaceutical compositions comprising:

(a) N-hydroxy-3-[4-[[[2-(2-methyl-1 H-indol-3-yl)-ethyl]-amino]methyl]phenyl]-2E-2-propenamide;

(b) a pharmaceutically active agent; and

(c) a pharmaceutically acceptable carrier.

The present invention further relates to a commercial package or product comprising:

(a) a pharmaceutical formulation of N-hydroxy-3-[4-[[[2-(2-methyl-1 H-indol-3-yl)-ethyl]-amino]methyl]phenyl]-2E-2-propenamide; and

(b) a pharmaceutical formulation of a pharmaceutically active agent for simultaneous, concurrent, separate or sequential use.

The combination partners (a) and (b) can be administered together, one after the other or separately in one combined unit dosage form or in two separate unti dosage forms. The unit dosage form may also be a fixed combination.
The present invention further relates to a method of preventing or treating proliferative diseases or diseases that are associated with or triggered by persistent angiogenesis in a mammal, particularly a human, with a combination comprising:

(a) N-hydroxy-3-[4-[[[2-(2-methyl-1 H-indol-3-yl)-ethyl]-amino]methyl]phenyl]-2E-2-propenamide; and

(b) one or more pharmaceutically active agents.

## Detailed Description of the Invention

N-hydroxy-3-[4-[[[2-(2-methyl-1 H-indol-3-yl)-ethyl]-amino]methyl]phenyl]-2E-2-propenamide is a HDAI and has the following structure:

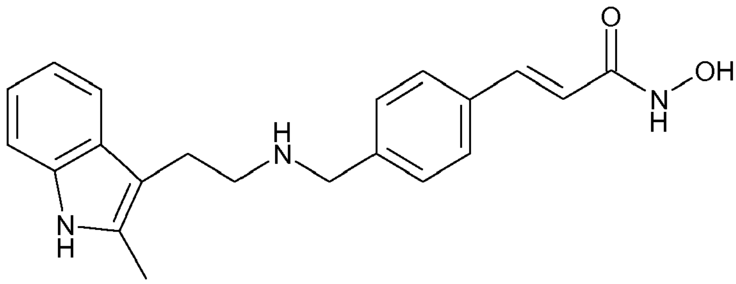

.

The term "pharmaceutically active agents" is a broad one covering many pharmaceutically active agents having different mechanisms of action. Combinations of some of these with N-hydroxy-3-[4-[[[2-(2-methyl-1H-indol-3-yl)-ethyl]-amino]methyl]phenyl]-2E-2-propenamide can result in improvements in cancer therapy. Generally, pharmaceutically active agents are classified according to the mechanism of action. Many of the available agents are anti-metabolites of development pathways of various tumors, or react with the DNA of the tumor cells. There are also agents which inhibit enzymes, such as topoisomerase I and topoisomerase II, or which are antimiotic agents.

By the term "pharmaceutically active agent" is meant especially any pharmaceutically active agent other than N-hydroxy-3-[4-[[[2-(2-methyl-1H-indol-3-yl)-ethyl]-amino]methyl]phenyl]-2E-2-propenamide or a derivative thereof. It includes, but is not limited to:

i. an ACE inhibitor;
ii. an adenosine-kinase-inhibitor;
iii. an adjuvant;
iv. an adrenal cortex antagonist;
v. AKT pathway inhibitor;
vi. an alkylating agent;
vii. an angiogenesis inhibitor;
viii. an angiostatic steroid;
ix. an anti-androgen;
x. an anti-estrogen;
xi. an anti-hypercalcemia agent;
xii. an anti-leukemic compound;
xiii. an anti-metabolite;
xiv. an anti-proliferative antibody;
xv. an apoptosis inducer;
xvi. an AT1 receptor antagonist;
xvii. an aurora kinase inhibitor;
xviii. an aromatase inhibitor;
xix. a biological response modifier;
xx. a bisphosphonate;
xxi. a Bruton’s Tyrosine Kinase (BTK) inhibitor;
xxii. a calcineurin inhibitor;
xxiii. a CaM kinase II inhibitor;
xxiv. a CD45 tyrosine phosphatase inhibitor;
xxv. a CDC25 phosphatase inhibitor;
xxvi. a CYP3A4 inhibitor;
xxvii. a CHK kinase inhibitor;
xxviii. a compound targeting/decreasing a protein or lipid kinase activity or a protein or lipid phosphatase activity, a further anti-angiogenic compound or a compound which induces cell differentiation processes;
xxix. a controlling agent for regulating genistein, olomucine and/or tyrphostins;
xxx. a cyclooxygenase inhibitor;
xxxi. a cRAF kinase inhibitor;
xxxii. a cyclin dependent kinase inhibitor;
xxxiii. a cysteine protease inhibitor;
xxxiv. a DNA intercalator;
xxxv. a DNA strand breaker;

xxxvi. an E3 Ligase inhibitor;
xxxvii. an EDG binder;
xxxviii. an endocrine hormone;
xxxix. compounds targeting, decreasing or inhibiting the activity of the epidermal growth factor family;
xl. an EGFR, PDGFR tyrosine kinase inhibitor;
xli. a farnesyltransferase inhibitor;
xlii. a Flk-1 kinase inhibitor;
xliii. a compound which targets, decreases or inhibits the activity of Flt-3;
xliv. a gonadorelin agonist;
xlv. a Glycogen synthase kinase-3 (GSK3) inhibitor;
xlvi. a heparanase inhibitor;
xlvii. an agent used in the treatment of hematologic malignancies;
xlviii. a histone deacetylase (HDAC) inhibitor;
xlix. a HSP90 inhibitor;
l. an implant containing corticosteroids;
li. a I-kappa B-alpha kinase inhibitor (IKK);
lii. an insulin receptor tyrosine kinase inhibitor;
liii. a c-Jun N-terminal kinase (JNK) kinase inhibitor;
liv. a microtubule binding agent;
lv. a Mitogen-activated protein (MAP) kinase-inhibitor;
lvi. a MDM2 inhibitor;
lvii. a MEK inhibitor;
lviii. a methionine aminopeptidase inhibitor;
lix. a matrix metalloproteinase inhibitor (MMP) inhibitor;
lx. a monoclonal antibody;
lxi. a NGFR tyrosine-kinase-inhibitor;
lxii. a p38 MAP kinase inhibitor, including a SAPK2/p38 kinase inhibitor;
lxiii. a p56 tyrosine kinase inhibitor;
lxiv. a PDGFR tyrosine kinase inhibitor;
lxv. a phosphatidylinositol 3-kinase inhibitor;
lxvi. a phosphatase inhibitor;
lxvii. photodynamic therapy;
lxviii. a platinum agent;
lxix. a protein phosphatase inhibitor, including a PP1 and PP2 inhibitor and a tyrosine phosphatase inhibitor;
lxx. a PKC inhibitor and a PKC delta kinase inhibitor;
lxxi. a polyamine synthesis inhibitor;
lxxii. a proteosome inhibitor;
lxxiii. a PTP1B inhibitor;
lxxiv. a protein tyrosine kinase inhibitor including a SRC family tyrosine kinase inhibitor; a Syk tyrosine kinase inhibitor; and a JAK-2 and/or JAK-3 tyrosine kinase inhibitor;
lxxv. an inhibitor of Ras oncogenic isoforms;
lxxvi. a retinoid;
lxxvii. a ribonucleotide reductase inhibitor;
lxxviii. a RNA polymerase II elongation inhibitor;
lxxix. an S-adenosylmethionine decarboxylase inhibitor;
lxxx. a serine/threonine kinase inhibitor;
lxxxi. a compound which targets, decreases or inhibits the activity/function of serine/theronine mTOR kinase;
lxxxii. a somatostatin receptor antagonist;
lxxxiii. a sterol biosynthesis inhibitor;
lxxxiv. a telomerase inhibitor;
lxxxv. a topoisomerase inhibitor;
lxxxvi. tumor cell damaging approaches;
lxxxvii. a monoclonal antibody of VEGF or VEGFR;
lxxxviii. VEGFR tyrosine kinase inhibitor; and
lxxxix. a RANKL Inhibitor.

The term "ACE inhibitor", as used herein, includes, but is not limited to, CIBACEN, benazepril, enazepril (LOTENSIN), captopril, enalapril, fosinopril, lisinopril, moexipril, quinapril, ramipril, perindopril and trandolapril.

The term "an adenosine-kinase-inhibitor", as used herein, relates to a compound which targets, decreases or inhibits nucleobase, nucleoside, nucleotide and nucleic acid metabolisms. An example of an adenosine-kinase-inhibitor includes, but is not limited to, 5-Iodotubercidin, which is also known as 7H-pyrrolo[2,3-d]pyrimidin-4-amine, 5-iodo-7-β-D-ribofuranosyl-(9Cl).

The term "an adjuvant", as used herein, refers to a compound which enhances the 5-FU-TS bond, as well as a compound which targets, decreases or inhibits, alkaline phosphatase. Examples of an adjuvant include, but are not limited to, Leucovorin and Levamisole.

The term "an adrenal cortex antagonist", as used herein, relates to a compound which targets, decreases or inhibits the activity of the adrenal cortex and changes the peripheral metabolism of corticosteroids, resulting in a decrease in 17-hydroxycorticosteroids. An example of an adrenal cortex antagonist includes, but is not limited to, Mitotane.

The term "AKT pathway inhibitor", as used herein, relates to a compound which targets, decreases or inhibits cell proliferation. Akt, also known as protein kinase B (PKB), a serine/threonine kinase, is a critical enzyme in several signal transduction pathways involved in diabetes. The principal role of Akt in the cell is to facilitate growth factor-mediated cell survival and to block apoptotic cell death. A target of the AKT pathway inhibitor includes, but is not limited to, Pi3K/AKT. Examples of an AKT pathway inhibitor include, but are not limited to, Deguelin, which is also known as 3H-bis[1]benzopyrano[3,4-b:6',5'-e]pyran-7(7aH)-one, 13, 13a-dihydro-9,10-dimethoxy-3,3-dimethyl-, (7aS, 13aS)-(9Cl); and Trciribine, which is also known as 1,4,5,6,8-pentaazaacenaphthylen-3-amine, 1,5-dihydro-5-methyl-1-β-D-ribofuranosyl-(9Cl).

The term "an alkylating agent", as used herein, relates to a compound which causes alkylation of DNA and results in breaks in the DNA molecules, as well as cross-linking of the twin strands, thus interfering with DNA replication and transcription of RNA. Examples of an alkylating agent include, but are not limited to, Chlorambucil, cyclophosphamide, Dacarbazine, Lomustine, Procarbazine, Thiotepa, Melphalan, Temozolomide (TEMODAR), Carmustine, Ifosfamide, Mitomycin, Altretamine, Busulfan, Machlorethamine hydrochloride, nitrosourea (BCNU or Gliadel), Streptozocin, and estramustine. Cyclophosphamide can be administered, e.g., in the form as it is marketed, e.g., under the trademark CYCLOSTIN; and ifosfamide as HOLOXAN.

The term "an angiogenesis inhibitor", as used herein, relates to a compound which targets, decreases or inhibits the production of new blood vessels. Targets of an angiogenesis inhibitor include, but are not limited to, methionine aminopeptidase-2 (MetAP-2), macrophage inflammatory protein-1 (MIP-1α), CCL5, TGF-β, lipoxygenase, cyclooxygenase, and topoisomerase. Indirect targets of an angiogenesis inhibitor include, but are not limited to, p21, p53, CDK2 and collagen synthesis. Examples of an angiogenesis inhibitor include, but are not limited to, Fumagillin, which is known as 2,4,6,8-decatetraenedioic acid, mono[(3R,4S,5S,6R)-5-methoxy-4-[(2R,3R)-2-methyl-3-(3-methyl-2-butenyl)oxiranyl]-1-oxaspiro[2.5]oct-6-yl] ester, (2E,4E,6E,8E)-(9Cl); Shikonin, which is also known as 1,4-naphthalenedione, 5,8-dihydroxy-2-[(1R)-1-hydroxy-4-methyl-3-pentenyl]-(9Cl); Tranilast, which is also known as benzoic acid, 2-[[3-(3,4-dimethoxyphenyl)-1-oxo-2-propenyl]amino]-(9Cl); ursolic acid; suramin; thalidomide and lenalidomide, and marketed as REVLIMID.

The term "angiostatic steroid", as used herein, includes, but is not limited to, agents which block or inhibit angiogenesis, such as, e.g., anecortave, triamcinolone, hydrocortisone, 11-α-epihydrocotisol, cortexolone, 17α-hydroxyprogesterone, corticosterone, desoxycorticosterone, testosterone, estrone and dexamethasone.

The term "an anti-androgen", as used herein, relates to a compound which blocks the action of androgens of adrenal and testicular origin which stimulate the growth of normal and malignant prostatic tissue. Examples of an anti-androgen include, but are not limited to, Nilutamide; bicalutamide (CASODEX), which can be formulated, e.g., as disclosed in U.S. Patent No. 4,636,505.

The term "an anti-estrogen", as used herein, relates to a compound which antagonizes the effect of estrogens at the estrogen receptor level. Examples of an anti-estrogen include, but are not limited to, Toremifene; Letrozole; Testolactone; Anastrozole; Bicalutamide; Flutamide; Tamoxifen Citrate; Exemestane; Fulestrant; tamoxifen; fulvestrant; raloxifene and raloxifene hydrochloride. Tamoxifen can be administered in the form as it is marketed, e.g., NOLVADEX; and raloxifene hydrochloride is marketed as EVISTA. Fulvestrant can be formulated as disclosed in U.S. Patent No. 4,659,516 and is marketed as FASLODEX. A combination of the invention comprising a pharmaceutically active agent which is an anti-estrogen is particularly useful for the treatment of estrogen receptor positive tumors, e.g., breast tumors.

The term "an anti-hypercalcemia agent", as used herein, refers to compounds which are used to treat hypercalcemia. Examples of an anti-hypercalcemia agent include, but are not limited to, gallium (III) nitrate hydrate; and pamidronate disodium.

The term "anti-leukemic compound", as used herein, includes, but is not limited to, Ara-C, a pyrimidine analog, which is the 2'-α-hydroxy ribose (arabinoside) derivative of deoxycytidine. Also included is the purine analog of hypoxanthine, 6-mercaptopurine (6-MP) and fludarabine phosphate.

The term "an anti-metabolite", as used herein, relates to a compound which inhibits or disrupts the synthesis of DNA resulting in cell death. Examples of an antimetabolite include, but are not limited to, 6-mercaptopurine; Cytarabine; Fludarabine; Flexuridine; Fluorouracil; Capecitabine; Raltitrexed; Methotrexate; Cladribine; Gemcitabine; Gemcitabine

hydrochloride; Thioguanine; Hydroxyurea; DNA de-methylating agents, such as 5-azacytidine and decitabine; edatrexate; and folic acid antagonists such as, but not limited to, pemetrexed. Capecitabine can be administered, e.g., in the form as it is marketed, e.g., under the trademark XELODA; and gemcitabine as GEMZAR.

The term "an antiproliferative antibody", as used herein, includes, but is not limited to, trastuzumab, trastuzumab-DM1, erlotinib (TARCEVA), Panitumumab, bevacizumab (AVASTIN), rituximab (RITUXAN), PRO64553 (anti-CD40) and 2C4 Antibody. By antibodies is meant, e.g., intact monoclonal antibodies, polyclonal antibodies, multispecific antibodies formed from at least two intact antibodies, and antibodies fragments so long as they exhibit the desired biological activity.

The term "an apoptosis inducer", as used herein, relates to a compound which induces the normal series of events in a cell that leads to its death. The apoptosis inducer of the present invention may selectively induce the X-linked mammalian inhibitor of apoptosis protein XIAP. The apoptosis inducer of the present invention may downregulate BCL-xL. Examples of an apoptosis inducer include, but are not limited to, ethanol, 2-[[3-(2,3-dichlorophenoxy)propyl]amino]-(9Cl); gambogic acid; Embelin, which is also known as 2,5-cyclohexadiene-1,4-dione, 2,5-dihydroxy-3-undecyl-(9Cl); and Arsenic Trioxide.

The term "AT1 receptor antagonist", as used herein, includes, but is not limited to, agents, such as DIOVAN.

The term "an aurora kinase inhibitor", as used herein, relates to a compound which targets, decreases or inhibits later stages of the cell cycle from the G2/M check point all the way through to the mitotic checkpoint and late mitosis. An example of an aurora kinase inhibitor includes, but is not limited to, Binucleine 2, which is also known as Methanimidamide, N'-[1-(3-chloro-4-fluorophenyl)-4-cyano-1H-pyrazol-5-yl]-N,N-dimethyl-(9Cl).

The term "aromatase inhibitor", as used herein, relates to a compound which inhibits the estrogen production, i.e., the conversion of the substrates androstenedione and testosterone to estrone and estradiol, respectively. The term includes, but is not limited to, steroids, especially atamestane, exemestane and formestane; and, in particular, non-steroids, especially aminoglutethimide, roglethimide, pyridoglutethimide, trilostane, testolactone, ketokonazole, vorozole, fadrozole, anastrozole and letrozole. Exemestane is marketed as AROMASIN; formestane as LENTARON; fadrozole as AFEMA; anastrozole as ARIMIDEX; letrozole as FEMARA or FEMAR; and aminoglutethimide as ORIMETEN. A combination of the invention comprising a pharmaceutically active agent which is an aromatase inhibitor is particularly useful for the treatment of hormone receptor positive tumors, e.g., breast tumors.

The term "biological response modifier", as used herein, includes, but is not limited to, lymphokine or interferons, e.g., interferon $\gamma$.

The term "bisphosphonates", as used herein, includes, but is not limited to, etridonic, clodronic, tiludronic, pamidronic, alendronic, ibandronic, risedronic and zoledronic acid. "Etridonic acid" can be administered, e.g., in the form as it is marketed, e.g., DIDRONEL; "clodronic acid" as BONEFOS; "tiludronic acid" as SKELID; "pamidronic acid" as AREDIA; "alendronic acid" as FOSAMAX; "ibandronic acid" as BONDRANAT; "risedronic acid" as ACTONEL; and "zoledronic acid" as ZOMETA.

The term "a Bruton's Tyrosine Kinase (BTK) inhibitor", as used herein, relates to a compound which targets, decreases or inhibits human and murine B cell development. An example of a BTK inhibitor includes, but is not limited to, terreic acid.

The term "a calcineurin inhibitor", as used herein, relates to a compound which targets, decreases or inhibits the T cell activation pathway. A target of a calcineurin inhibitor includes protein phosphatase 2B. Examples of a calcineurin inhibitor include, but are not limited to, Cypermethrin, which is also known as cyclopropanecarboxylic acid, 3-(2,2-dichloroethenyl)-2,2-dimethyl-,cyano(3-phenoxyphenyl)methyl ester (9Cl); Deltamethrin, which is also known as cyclopropanecarboxylic acid, 3-(2,2-dibromoethenyl)-2,2-dimethyl-(S)-cyano(3-phenoxyphenyl)methyl ester, (1R,3R)-(9Cl); Fenvalerate, which is also known as benzeneacetic acid, 4-chloro-$\alpha$-(1-methylethyl)-,cyano(3-phenoxyphenyl)methyl ester (9Cl); and Tyrphostin 8.

The term "a CaM kinase II inhibitor", as used herein, relates to a compound which targets, decreases or inhibits CaM Kinases. CaM Kinases constitute a family of structurally related enzymes that include phosphorylase kinase, myosin light chain kinase, and CaM kinases I-IV. CaM Kinase II, one of the best-studied multifunctional enzymes, is found in high concentrations in neuronal synapses, and in some regions of the brain it may constitute up to 2% of the total protein content. Activation of CaM kinase II has been linked to memory and learning processes in the vertebrate nervous system. Targets of a CaM kinase II inhibitor include CaM kinase II. Examples of a CaM kinase II inhibitor include, but are not limited to, 5-isoquinolinesulfonic acid, 4-[(2S)-2-[(5-isoquinolinylsulfonyl)methylamino]-3-oxo-3-(4-phenyl-1-piperazinyl) propyl]phenyl ester (9Cl); and benzenesulfonamide, N-[2-[[[3-(4-chlorophenyl)-2-propenyl]methyl]amino]methyl]phenyl]-N-(2-hydroxyethyl)-4-methoxy-(9Cl).

The term "a CD45 tyrosine phosphatase inhibitor", as used herein, relates to a compound which targets, decreases or inhibits dephosphorylating regulatory pTyr residues on SRC-family protein-tyrosine kinases, which aids in the treatment of a variety of inflammatory and immune disorders. An example of a CD45 tyrosine phosphatase inhibitor includes, but is not limited to, Phosphonic acid, [[2-(4-bromophenoxy)-5-nitrophenyl]hydroxymethyl]-(9Cl).

The term "a CDC25 phosphatase inhibitor", as used herein, relates to compound which targets, decreases or

inhibits overexpressed dephosphorylate cyclin-dependent kinases in tumors. An example of a CDC25 phosphatase inhibitor includes 1,4-naphthalenedione, 2,3-bis[(2-hydroyethyl)thio]-(9Cl).

The term "a CHK kinase inhibitor", as used herein, relates to a compound which targets, decreases or inhibits overexpression of the antiapoptotic protein Bcl-2. Targets of a CHK kinase inhibitor are CHK1 and/or CHK2. An example of a CHK kinase inhibitor includes, but is not limited to, Debromohymenialdisine.

The term "compounds targeting/decreasing a protein or lipid kinase activity; or a protein or lipid phosphatase activity; or further anti-angiogenic compounds", as used herein, includes, but is not limited to, protein tyrosine kinase and/or serine and/or theroine kinase inhibitors or lipid kinase inhibitors, e.g.,

i) compounds targeting, decreasing or inhibiting the activity of the vascular endothelial growth factor-receptors (VEGF), such as compounds which target, decrese or inhibit the activity of VEGF, especially compounds which inhibit the VEGF receptor, such as, but not limited to, 7H-pyrrolo[2,3-d]pyrimidine derivatives, including {6-[4-(4-ethyl-piperazine-1-yimethyl)-phenyl]-7H-pyrrolo[2,3-d]pyrinidinpyrimidin-4-yl]-((R)-1-phenyl-ethyl)-amine (known as AEE788); BAY 43-9006; isolcholine compounds disclosed in WO 00/09495, such as (4-tert-butyl-phenyl)-94-pyridin-4-ylmethyl-isoquinolin-1-yl)-amine;
ii) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g., a N-phenyl-2-pyrimidine-amine derivative, e.g., imatinib, SU101, SU6668 and GFB-111;
iii) compounds targeting, decreasing or inhibiting the activity of the fibroblast growth factor-receptors (FGFR);
iv) compounds targeting, decreasing or inhibiting the activity of the insulin-like growth factor receptor 1 (IGF-1R), such as compounds which target, decrease or inhibit the activity of IGF-IR, especially compounds which inhibit the IGF-1 R receptor. Compounds include, but are not limited to, the compounds disclosed in WO 02/092599 and derivatives thereof of 4-amino-5-phenyl-7-cyclobutyl-pyrrolo[2,3-d]pyrimidine derivatives;
v) compounds targeting, decreasing or inhibiting the activity of the Trk receptor tyrosine kinase family;
vi) compounds targeting, decreasing or inhibiting the activity of the Axl receptor tyrosine kinase family;
vii) compounds targeting, decreasing or inhibiting the activity of the c-Met receptor;
viii) compounds targeting, decreasing or inhibiting the activity of the Ret receptor tyrosine kinase;
ix) compounds targeting, decreasing or inhibiting the activity of the Kit/SCFR receptor tyrosine kinase;
x) compounds targeting, decreasing or inhibiting the activity of the C-kit receptor tyrosine kinases (part of the PDGFR family), such as compounds which target, decrease or inhibit the activity of the c-Kit receptor tyrosine kinase family, especially compounds which inhibit the c-Kit receptor, e.g., imatinib;
xi) compounds targeting, decreasing or inhibiting the activity of members of the c-Abl family and their gene-fusion products, e.g., BCR-Abl kinase, such as compounds which target decrease or inhibit the activity of c-Abl family members and their gene fusion products, e.g., a N-phenyl-2-pyrimidine-amine derivative, e.g., imatinib, PD180970, AG957, NSC 680410 or PD173955 from ParkeDavis; BMS354825;
xii) compounds targeting, decreasing or inhibiting the activity of members of the protein kinase C (PKC) and Raf family of serine/threonine kinases, members of the MEK, SRC, JAK, FAK, PDK and Ras/MAPK family members, or PI(3) kinase family, or of the PI(3)-kinase-related kinase family, and/or members of the cyclin-dependent kinase family (CDK) and are especially those staurosporine derivatives disclosed in U.S. Patent No. 5,093,330, e.g., midostaurin; examples of further compounds include, e.g., UCN-01; safingol; BAY 43-9006; Bryostatin 1; Perifosine; Ilmofosine; RO 318220 and RO 320432; GO 6976; Isis 3521; LY333531/LY379196; isochinoline compounds, such as those disclosed in WO 00/09495; FTIs; PD184352 or QAN697, a P13K inhibitor;
xiii) compounds targeting, decreasing or inhibiting the activity of protein-tyrosine kinase, such as imatinib mesylate (GLEEVEC); tyrphostin or pyrymidylaminobenzamide and derivatives thereof. A tyrphostin is preferably a low molecular weight ($M_r$ <1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810, AG 99, Tyrphostin AG 213, Tyrphostin AG 1748, Tyrphostin AG 490, Tyrphostin B44, Tyrphostin B44 (+) enantiomer, Tyrphostin AG 555, AG 494, Tyrphostin AG 556; AG957 and adaphostin (4-{[(2,5-dihydroxyphenyl)methyl]aminol-benzoic acid adamantyl ester, NSC 680410, adaphostin);
xiv) compounds targeting, decreasing or inhibiting the activity of the epidermal growth factor family of receptor tyrosine kinases (EGFR, ErbB2, ErbB3, ErbB4 as homo-or heterodimers), such as compounds which target, decrease or inhibit the activity of the epidermal growth factor receptor family are especially compounds, proteins or antibodies which inhibit members of the EGF receptor tyrosine kinase family, e.g., EGF receptor, ErbB2, ErbB3 and ErbB4 or bind to EGF or EGF-related ligands, and are in particular those compounds, proteins or monoclonal antibodies generically and specifically disclosed in WO 97/02266, e.g., the compound of Example 39, or in EP 0 564 409, WO 99/03854, EP 0520722, EP 0 566 226, EP 0 787 722, EP 0 837 063, U.S. Patent No. 5,747,498, WO 98/10767, WO 97/30034, WO 97/49688, WO 97/38983 and, especially, WO 96/30347, e.g., compound known as

CP 358774, WO 96/33980, e.g., compound ZD 1839; and WO 95/03283, e.g., compound ZM105180, e.g., trastuzumab (HERCEPTIN®), cetuximab, Iressa, OSI-774, CI-1033, EKB-569, Lapatinib, E1.1, E2.4, E2.5, E6.2, E6.4, E2.11, E6.3 or E7.6.3, and 7H-pyrrolo-[2,3-d]pyrimidine derivatives which are disclosed in WO 03/013541, erlotinib and gefitinib. Erlotinib can be administered in the form as it is marketed, e.g. TARCEVA, and gefitinib as IRESSA, human monoclonal antibodies against the epidermal growth factor receptor including ABX-EGFR; and xv) compounds which target, decrease or inhibit the activity/function of serine/theronine mTOR kinase are especially compounds, proteins or antibodies which target/inhibit members of the mTOR kinase family, e.g., RAD, RAD001, CCI-779, ABT578, SAR543, rapamycin and derivatives/analogs thereof, AP23573 and AP23841 from Ariad, everolimus (CERTICAN) and sirolimus. CERTICAN (everolimus, RAD) an investigational novel proliferation signal inhibitor that prevents proliferation of T-cells and vascular smooth muscle cells.

When referring to antibody, it is to include intact monoclonal antibodies, nanobodies, polyclonal antibodies, multi-specific antibodies formed from at least 2 intact antibodies, and antibodies fragments so long as they exhibit the desired biological activity.

The phrase "compound which targets, decreases or inhibits the activity of a protein or lipid phosphatase", as used herein, includes, but is not limited to, inhibitors of phosphatase 1, phosphatase 2A, PTEN or CDC25, e.g., okadaic acid or a derivative thereof.

The phrase "further anti-angiogenic compounds" includes, but is not limited to, compounds having another mechanism for their activity, e.g., unrelated to protein or lipid kinase inhibition, e.g., thalidomide (THALOMID), lenalidomide (REVLIMID) and TNP-470.

The phrase "compounds which induce cell differentiation processes", as used herein, includes, but is not limited to, retinoic acid, α-, γ- or δ-tocopherol or α-, γ- or δ-tocotrienol.

Examples of a "controlling agent for regulating genistein, olomucine and/or tyrphostins" includes, but are not limited to, Daidzein, which is also known as 4H-1-benzopyran-4-one, 7-hydroxy-3-(4-hydroxyphenyl)-(9Cl); Iso-Olomoucine, and Tyrphostin 1.

The term "cyclooxygenase inhibitor", as used herein, includes, but is not limited to, e.g., Cox-2 inhibitors. The term "a COX-2 inhibitor", as used herein, relates to a compound which targets, decreases or inhibits the enzyme cox-2 (cyclooxygenase-2). Examples of a COX-2 inhibitor include, but are not limited to, 1 H-indole-3-acetamide, 1-(4-chlorobenzoyl)-5-methoxy-2-methyl-N-(2-phenylethyl)-(9Cl); 5-alkyl substituted 2-arylaminophenylacetic acid and derivatives, such as celecoxib (CELEBREX), rofecoxib (VIOXX), etoricoxib, valdecoxib; or a 5-alkyl-2-arylaminophenylacetic acid, e.g., 5-methyl-2-(2'-chloro-6'-fluoroanilino)phenyl acetic acid, lumiracoxib; and celecoxib.

The term "a cRAF kinase inhibitor", as used herein, relates to a compound which targets, decreases or inhibits the up-regulation of E-selectin and vascular adhesion molecule-1 induced by TNF. Raf kinases play an important role as extracellular signal-regulating kinases in cell differentiation, proliferation and apoptosis. A target of a cRAF kinase inhibitor includes, but is not limited, to RAF1. Examples of a cRAF kinase inhibitor include, but are not limited to, 3-(3,5-dibromo-4-hydroxybenzylidene)-5-iodo-1,3-dihydroindol-2-one; and benzamide, 3-(dimethylamino)-N-[3-[(4-hydroxybenzoyl)amino]-4-methylphenyl]-(9Cl).

The term "a cyclin dependent kinase inhibitor", as used herein, relates to a compound which targets, decreases or inhibits cyclin dependent kinase which play a role in the regulation of the mammalian cell cycle. Cell cycle progression is regulated by a series of sequential events that include the activation and subsequent inactivation of cyclin dependent kinases (Cdks) and cyclins. Cdks are a group of serine/threonine kinases that form active heterodimeric complexes by binding to their regulatory subunits, cyclins. Examples of targets of a cyclin dependent kinase inhibitor include, but are not limited to, CDK, AHR, CDK1, CDK2, CDK5, CDK4/6, GSK3beta and ERK. Examples of a cyclin dependent kinase inhibitor include, but are not limited to, N9-Isopropyl-Olomoucine; Olomoucine; Purvalanol B, which is also known as Benzoic acid, 2-chloro-4-[[2-[[(1 R)-1-(hydroxymethyl)-2-methyipropyl]amino]-9-(1-methylethyl)-9H-purin-6-yl]amino]-(9Cl); Roascovitine; Indirubin, which is also known as 2H-Indol-2-one, 3-(1,3-dihydro-3-oxo-2H-indol-2-ylidene)-1,3-dihydro-(9Cl); Kenpaullone, which is also known as Indolo[3,2-d][1]benzazepin-6(5H)-one, 9-bromo-7,12-dihydro-(9Cl); purvalanol A, which is also known as 1-Butanol, 2-[[6-[(3-chlorophenyl)amino]-9-(1-methylethyl)-9H-purin-2-yl]amino]-3-methyl-, (2R)-(9Cl); and Indirubin-3'-monooxime.

The term "a cysteine protease inhibitor", as used herein, relates to a compound which targets, decreases or inhibits cystein protease which plays a vital role in mammalian cellular turnover and apoptosis. An example of a cystein protease inhibitor includes, but is not limited to, 4-morpholinecarboxamide, N-[(1S)-3-fluoro-2-oxo-1-(2-phenylethyl) propyl]amino]-2-oxo-1-(phenylmethyl)ethyl]-(9Cl).

The term "a DNA intercalator", as used herein, relates to a compound which binds to DNA and inhibits DNA, RNA and protein synthesis. Examples of a DNA intercalator include, but are not limited to, Plicamycin and Dactinomycin.

The term "a DNA strand breaker", as used herein, relates to a compound which causes DNA strand scission and results in inhibition of DNA synthesis, ininhibition of RNA and protein synthesis. An example of a DNA strand breaker includes, but is not limited to, Bleomycin.

The term "an E3 Ligase inhibitor", as used herein, relates to a compound which targets, decreases or inhibits the E3 ligase which inhibits the transfer of ubiquitin chains to proteins, marking them for degradation in the proteasome. An example of a E3 ligase inhibitor includes, but is not limited to, N-((3,3,3-trifluoro-2-trifluoromethyl)propionyl)sulfanilamide.

The term "EDG binder", as used herein, includes, but is not limited to, a class of immunosuppressants that modulates lymphocyte recirculation, such as FTY720.

The term "an endocrine hormone", as used herein, relates to a compound which by acting mainly on the pituitary gland causes the suppression of hormones in males, the net effect is a reduction of testosterone to castration levels. In females, both ovarian estrogen and androgen synthesis are inhibited. An example of an endocrine hormone includes, but is not limited to, Leuprolide and megestrol acetate.

The term "compounds targeting, decreasing or inhibiting the activity of the epidermal growth factor family", as used herein, relates to a compound which icompounds targeting, decreasing or inhibiting the activity of the epidermal growth factor family of receptor tyrosine kinases (EGFR, ErbB2, ErbB3, ErbB4 as homo- or heterodimers), such as compounds which target, decrease or inhibit the activity of the epidermal growth factor receptor family are especially compounds, proteins or antibodies which inhibit members of the EGF receptor tyrosine kinase family, e.g., EGF receptor, ErbB2, ErbB3 and ErbB4 or bind to EGF or EGF-related ligands, and are in particular those compounds, proteins or monoclonal antibodies generically and specifically disclosed in WO 97/02266, e.g., the compounds in EP 0 564 409, WO 99/03854, EP 0520722, EP 0 566 226, EP 0 787 722, EP 0 837 063, U.S. Patent No. 5,747,498, WO 98/10767, WO 97/30034, WO 97/49688, WO 97/38983 and, especially, WO 96/30347, e.g., compound known as CP 358774, WO 96/33980, e.g., compound ZD 1839; and WO 95/03283, e.g., compound ZM105180, e.g., trastuzumab (HERCEPTIN®), cetuximab, Iressa, OSI-774, CI-1033, EKB-569, Lapatinib, E1.1, E2.4, E2.5, E6.2, E6.4, E2.11, E6.3 or E7.6.3, and 7H-pyrrolo-[2,3-d]pyrimidine derivatives which are disclosed in WO 03/013541, erlotinib and gefitinib. Erlotinib can be administered in the form as it is marketed, e.g., TARCEVA, and gefitinib as IRESSA, human monoclonal antibodies against the epidermal growth factor receptor including ABX-EGFR. Targets of an EGFR kinase inhibitor include, but are not limited to, guanylyl cyclase (GC-C) and HER2. Other examples of an EGFR kinase inhibitor include, but are not limited to, Tyrphostin 23, Tyrphostin 25, Tyrphostin 47, Tyrphostin 51 and Tyrphostin AG 825. Targets of an EGFR tyrosine kinase inhibitor include EGFR, PTK and tubulin. Other examples of an EGFR tyrosine kinase inhibitor include, but are not limited to, 2-propenamide, 2-cyano-3-(3,4-dihydroxyphenyl)-N-phenyl-,(2E)-(9Cl); Tyrphostin Ag 1478; Lavendustin A; and 3-pyridineacetonitrile, α-[(3,5-dichlorophenyl)methylene]-, (αZ)-(9Cl). An example of an EGFR, PDGFR tyrosine kinase inhibitor includes, but is not limited to, Tyrphostin 46.

The term "a farnesyltransferase inhibitor", as used herein, relates to a compound which targets, decreases or inhibits the Ras protein, which is commonly abnormally active in cancer. A target of a farnesyltransferase inhibitor includes, but is not limited to, RAS. Examples of a farnesyltransferase inhibitor include, but are not limited to, α-hydroxyfarnesylphosphonic acid; butanoic acid, 2-[[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl)-, 1-methylethyl ester, (2S)-(9cl); and Manumycin A.

The term "a Flk-1 kinase inhibitor", as used herein, relates to a compound which targets, decreases or inhibits Flk-1 tyrosine kinase activity. A target of a Flk-1 kinase inhibitor includes, but is not limited to, KDR. An example of a Flk-1 kinase inhibitor includes, but is not limited to, 2-propenamide, 2-cyano-3-[4-hydroxy-3,5-bis(1-methylethyl)phenyl]-N-(3-phenylpropyl)-,(2E)-(9Cl). The phrase "compounds which target, decrease or inhibit the activity of Flt-3", as used herein, includes, but is not limited to, compounds, proteins or antibodies which inhibit Flt-3, e.g., N-benzoyl-staurosporine, midostaurin, a staurosporine derivative, SU11248, also known as Sunitinib and is marketed as SUTENT, and MLN518.

The term "gonadorelin agonist", as used herein, includes, but is not limited to, abarelix, goserelin and goserelin acetate. Goserelin is disclosed in U.S. Patent No. 4,100,274 and is marketed as ZOLADEX. Abarelix can be formulated, e.g., as disclosed in U.S. Patent No. 5,843,901.

The term "a Glycogen synthase kinase-3 (GSK3) inhibitor", as used herein, relates to a compound which targets, decreases or inhibits glycogen synthase kinase-3 (GSK3). Glycogen Synthase Kinase-3 (GSK-3; tau protein kinase I), a highly-conserved, ubiquitously expressed serine/threonine protein kinase, is involved in the signal transduction cascades of multiple cellular processes, which is a protein kinase that has been shown to be involved in the regulation of a diverse array of cellular functions, including protein synthesis, cell proliferation, cell differentiation, microtubule assembly/disassembly and apoptosis. An example of a GSK3 inhibitor includes, but is not limited to, indirubin-3'-monooxime.

The term "heparanase inhibitor", as used herein, refers to compounds which target, decrease or inhibit heparin sulphate degradation. The term includes, but is not limited to, PI-88.

The phrase "agent used in the treatment of hematologic malignancies", as used herein, includes, but is not limited to, FMS-like tyrosine kinase inhibitors, e.g., compounds targeting, decreasing or inhibiting the activity of FMS-like tyrosine kinase receptors (Flt-3R); interferon, 1-b-D-arabinofuransylcytosine (ara-c) and bisulfan; and ALK inhibitors, e.g., compounds which target, decrease or inhibit anaplastic lymphoma kinase.

The term "a histone deacetylase (HDAC) inhibitor", as used herein, relates to a compound which inhibits the histone deacetylase and which possess anti-proliferative activity. This includes but is not limited to compounds disclosed in WO 02/22577, especially N-hydroxy-3-[4-[[(2-hydroxyethyl)[2-(1H-indol-3-yl)ethyl]-amino]methyl]phenyl]-2E-2-propenamide. It further includes suberoylanilide hydroxamic acid (SAHA); [4-(2-aminophenylcarbamoyl)-benzyl]-carbamic acid pyridine-3-ylmethyl ester and derivatives thereof; butyric acid, pyroxamide, trichostatin A, Oxamflatin, apicidin, Depsipeptide; depudecin and trapoxin. Other examples include depudecin; HC Toxin, which is also known as Cyclo[L-alanyl-D-alanyl-(αS,2S)-α-amino-η-oxooxiraneoctanoyl-D-prolyl] (9Cl); sodium phenylbutyrate, suberoyl bis-hydroxamic acid; and Trichostatin A.

The term "HSP90 inhibitor", as used herein, relates to a compound which targets, decreases or inhibits the intrinsic ATPase activity of HSP90; degrades, targets, decreases or inhibits the HSP90 client proteins via the ubiquitin proteosome pathway. Potential indirect targets of an HSP90 inhibitor include FLT3, BCR-ABL, CHK1, CYP3A5*3 and/or NQ01*2. Compounds targeting, decreasing or inhibiting the intrinsic ATPase activity of HSP90 are especially compounds, proteins or antibodies which inhibit the ATPase activity of HSP90, e.g., 17-allylamino,17-demethoxygeldanamycin (17AAG), a geldanamycin derivative; other geldanamycin-related compounds; radicicol and HDAC inhibitors. Other examples of an HSP90 inhibitor include geldanamycin,17-demethoxy-17-(2-propenylamino)-(9Cl); 5-(2,4-Dihydroxy-5-isopropyl-phenyl)-4-(4-morpholin-4-ylmethyl-phenyl)-isoxazole-3-carboxylic acid ethylamide; and Geldanamycin.

The phrase "an implant containing corticosteroids", as used herein, includes, but is not limited to, agents, such as, e.g., fluocinolone and dexamethasone.

The term "a I-kappa B-alpha kinase inhibitor (IKK)", as used herein, relates to a compound which targets, decreases or inhibits NF-kappaB. An example of an IKK inhibitor includes, but is not limited to, 2-propenenitrile, 3-[(4-methylphenyl)sulfonyl]-, (2E)-(9Cl).

The term "an insulin receptor tyrosine kinase inhibitor", as used herein, relates to a compound which modulates the activities of phosphatidylinositol 3-kinase, microtubule-associated protein, and S6 kinases. An example of an insulin receptor tyrosine kinase inhibitor includes, but is not limited to, hydroxyl-2-naphthalenylmethylphosphonic acid.

The term "a c-Jun N-terminal kinase (JNK) kinase inhibitor", as used herein, relates to a compound which targets, decreases or inhibits JNK. JNK, a serine-directed protein kinase, is involved in the phosphorylation and activation of c-Jun and ATF2 and plays a significant role in metabolism, growth, cell differentiation and apoptosis. A target for a JNK inhibitor includes, but is not limited to, DNMT. Examples of a JNK inhibitor include, but are not limited to, pyrazoleanthrone and/or epigallocatechin gallate.

The term "a microtubule binding agent", as used herein, refers to a compound which acts by disrupting the microtubular network that is essential for mitotic and interphase cellular function. Examples of a microtubule binding agent include, but are not limited to, Vinblastine Sulfate; Vincristine Sulfate; Vindesine; Vinorelbine; Docetaxel; Paclitaxel; vinorelbine; discodermolides; cochicine and epothilones and derivatives thereof, e.g., epothilone B or a derivative thereof. Paclitaxel is marketed as TAXOL; docetaxel as TAXOTERE; vinblastine sulfate as VINBLASTIN R.P; and vincristine sulfate as FARMISTIN. Also included are the generic forms of paclitaxel as well as various dosage forms of paclitaxel. Generic forms of paclitaxel include, but are not limited to, betaxolol hydrochloride. Various dosage forms of paclitaxel include, but are not limited to, albumin nanoparticle paclitaxel marketed as ABRAXANE; ONXOL, CYTOTAX. Discodermolide can be obtained, e.g., as disclosed in U.S. Patent No. 5,010,099. Also included are Epotholine derivatives which are disclosed in U.S. Patent No. 6,194,181, WO 98/10121, WO 98/25929, WO 98/08849, WO 99/43653, WO 98/22461 and WO 00/31247. Especially preferred are Epotholine A and/or B.

The term "a mitogen-activated protein (MAP) kinase inhibitor", as used herein, relates to a compound which targets, decreases or inhibits MAP. The MAP kinases are a group of protein serine/threonine kinases that are activated in response to a variety of extracellular stimuli and mediate signal transduction from the cell surface to the nucleus. They regulate several physiological and pathological cellular phenomena, including inflammation, apoptotic cell death, oncogenic transformation, tumor cell invasion and metastasis. An example of a MAP kinase inhibitor includes, but is not limited to, benzenesulfonamide, N-[2-[[[3-(4-chlorophenyl)-2-propenyl]methyl]amino]methyl]phenyl]-N-(2-hydroxyethyl)-4-methoxy-(9Cl).

The term "a MDM2 inhibitor", as used herein, relates to a compound which targets, decreases or inhibits the interaction of MDM2 and the p53 tumor suppressor. An example of a a MDM2 inhibitor includes, but is not limited to, trans-4-iodo, 4'-boranyl-chalcone.

The term "a MEK inhibitor", as used herein, relates to a compound which targets, decreases or inhibits the kinase activity of MAP kinase, MEK. A target of a MEK inhibitor includes, but is not limited to, ERK. An indirect target of a MEK inhibitor includes, but is not limited to, cyclin D1. An example of a MEK inhibitor includes, but is not limited to, butanedinitrile, bis[amino[2-aminophenyl)thio]methylene]-(9Cl).

The term "methionine aminopeptidase inhibitor", as used herein, includes, but is not limited to, compounds which target, decrease or inhibit the activity of methionine aminopeptidase. Compounds which target, decrease or inhibit the activity of methionine aminopeptidase are, e.g., bengamide or a derivative thereof.

The term "a MMP inhibitor", as used herein, relates to a compound which targets, decreases or inhibits a class

of protease enzyme that selectively catalyze the hydrolysis of polypeptide bonds including the enzymes MMP-2 and MMP-9 that are involved in promoting the loss of tissue structure around tumours and facilitating tumour growth, angiogenesis and metastasis. A target of a MMP inhibitor includes, but is not limited to, polypeptide deformylase. Example of a MMP inhibitor include, but are not limited to, Actinonin, which is also known as Butanediamide, N4-hydroxy-N1-[(1S)-1-[[(2S)-2-(hydroxymethyl)-1-pyrrolidinyl]carbonyl]-2-methylpropyl]-2-pentyl-, (2R)-(9Cl); epigallocatechin gallate; collagen peptidomimetic and non-peptidomimetic inhibitors; tetracycline derivatives, e.g., hydroxamate peptidomimetic inhibitor batimastat; and its orally-bioavailable analogue marimastat, prinomastat, metastat, Neovastat, Tanomastat, TAA211, MMI270B or AAJ996.

The term "monoclonal antibodies", as used herein, includes, but is not limited to, Panitumumab, bevacizumab, cetuximab, trastuzumab, Ibritumomab tiuxetan, and tositumomab and iodine I 131. Bevacizumab can be administered in the form as it is marketed, e.g., AVASTIN; cetuximab as ERBITUX; trastuzumab as HERCEPTIN; Rituximab as MABTHERA; Ibritumomab tiuxetan as ZEVULIN; and tositumomab and iodine I 131 as BEXXAR.

The term "a NGFR tyrosine-kinase-inhibitor", as used herein, relates to a compound which targets, decreases or inhibits nerve growth factor dependent $p140^{c\text{-}trk}$ tyrosine phosphorylation. Targets of a NGFR tyrosine-kinase-inhibitor include, but are not limited to, HER2, FLK1, FAK, TrkA and/or TrkC. An indirect target inhibits expression of RAF1. An example of a NGFR tyrosine-kinase-inhibitor includes, but is not limited to, Tyrphostin AG 879.

The term "a p38 MAP kinase inhibitor", as used herein, relates to a compound which targets, decreases or inhibits p38-MAPK, which is a MAPK family member. A MAPK family member is a serine/threonine kinase activated by phosphorylation of tyrosine and threonine residues. This kinase is phosphorylated and activated by many cellular stresses and inflammatory stimuli, thought to be involved in the regulation of important cellular responses, such as apoptosis and inflammatory reactions. An example of a a p38 MAP kinase inhibitor includes, but is not limited to, Phenol, 4-[4-(4-fluorophenyl)-5-(4-pyridinyl)-1 H-imidazol-2-yl]-(9Cl). An example of a a SAPK2/p38 kinase inhibitor includes, but is not limited to, benzamide, 3-(dimethylamino)-N-[3-[(4-hydroxybenzoyl)amino]-4-methylphenyl]-(9Cl).

The term "a p56 tyrosine kinase inhibitor", as used herein, relates to a compound which targets, decreases or inhibits p56 tyrosine kinase, which is an enzyme that is a lymphoid-specific SRC family tyrosine kinase critical for T-cell development and activation. A target of a p56 tyrosine kinase inhibitor includes, but is not limited to, Lck. Lck is associated with the cytoplasmic domains of CD4, CD8 and the beta-chain of the IL-2 receptor, and is thought to be involved in the earliest steps of TCR-mediated T-cell activation. Examples of a p56 tyrosine kinase inhibitor include, but are not limited to, damnacanthal, which is also known as 2-anthracenecarboxaldehyde,9,10-dihydro-3-hydroxy-1methoxy-9,10-dioxo-(9Cl), and/or Tyrphostin 46.

The term "a PDGFR tyrosine kinase inhibitor", as used herein, relates to compounds targeting, decreasing or inhibiting the activity of the C-kit receptor tyrosine kinases (part of the PDGFR family), such as compounds which target, decrease or inhibit the activity of the c-Kit receptor tyrosine kinase family, especially compounds which inhibit the c-Kit receptor, PDGF plays a central role in regulating cell proliferation, chemotaxis, and survival in normal cells, as well as in various disease states such as cancer, atherosclerosis and fibrotic disease. The PDGF family is composed of dimeric Isoforms (PDGF-AA, PDGF-BB, PDGF-AB, PDGF-CC, and PDGF-DD), which exert their cellular effects by differentially binding to two receptortyrosine kinases. PDGFR-$\alpha$ and PDGFR-$\beta$ have molecular masses of ~170 kDa and 180 kDa, respectively. Examples of targets of a PDGFR tyrosine kinase inhibitor includes, but are not limited to, PDGFR, FLT3 and/or C-kit. Example of a PDGFR tyrosine kinase inhibitor include, but are not limited to, Tyrphostin AG 1296; Tyrphostin 9; 1,3-butadiene-1,1,3-tricarbonitrile,2-amino-4-(1H-indol-5-yl)-(9Cl); Imatinib and IRESSA.

The term "a phosphatidylinositol 3-kinase inhibitor", as used herein, relates to a compound which targets, decreases or inhibits PI 3-kinase. PI 3-kinase activity has been shown to increase in response to a number of hormonal and growth factor stimuli, including insulin, platelet-derived growth factor, insulin-like growth factor, epidermal growth factor, colony-stimulating factor, and hepatocyte growth factor, and has been implicated in processes related to cellular growth and transformation. An example of a target of a phosphatidylinositol 3-kinase inhibitor includes, but is not limited to, Pi3K. Examples of a phosphatidylinositol 3-kinase inhibitor include, but are not limited to, Wortmannin, which is also known as 3H-furo[4,3,2-de]indeno[4,5-h]-2-benzopyran-3,6,9-trione, 11-(acetyloxy)-1,6b,7,8,9a,10,11,11 b-octahyd ro-1-(methoxymethyl)-9a,11b-dimethyl-, (1S,6bR,9aS,11R,11bR)- (9Cl); 8-phenyl-2-(morpholin-4-yl)-chromen-4-one; and/or Quercetin Dihydrate.

The term "a phosphatase inhibitor", as used herein, relates to a compound which targets, decreases or inhibits phosphatase. Phosphatases remove the phosphoryl group and restore the protein to its original dephosphorylated state. Hence, the phosphorylation-dephosphorylation cycle can be regarded as a molecular "on-off" switch. Examples of a phosphatase inhibitor include, but are not limited to, cantharidic acid; cantharidin; and L-leucinamide, N-[4-(2-carboxyethenyl)benzoyl]glycyl-L-$\alpha$-glutamyl-,(E)-(9Cl).

The term "photodynamic therapy", as used herein, refers to therapy which uses certain chemicals known as photosensitizing agents to treat or prevent cancers. Examples of photodynamic therapy include, but are not limited to, treatment with agents, such as, e.g., VISUDYNE and porfimer sodium.

The term "a platinum agent", as used herein, relates to a compound which contains Platinum and inhibit DNA

synthesis by forming interstrand and intrastrand cross-linking of DNA molecules. Examples of a a platinum agent include, but are not limited to, Carboplatin; Cisplatin; Oxaliplatin; cisplatinum; Satraplatin and platinum agents, such as ZD0473. Carboplatin can be administered, e.g., in the form as it is marketed, e.g., CARBOPLAT; and oxaliplatin as ELOXATIN.

The term "a protein phosphatase inhibitor", as used herein, relate to a compound which targets, decreases or inhibits protein phosphatase. The term "a PP1 or PP2 inhibitor", as used herein, relates to a compound which targets, decreases or inhibits Ser/Thr protein phosphatases. Type I phosphatases, which include PP1, can be inhibited by two heat-stable proteins known as Inhibitor-1 (I-1) and Inhibitor-2 (I-2). They preferentially dephosphorylate the P-subunit of phosphorylase kinase. Type II phosphatases are subdivided into spontaneously active (PP2A), $CA^{2+}$-dependent (PP2B), and $Mg^{2+}$-dependent (PP2C) classes of phosphatases. Examples of a PP1 and PP2A inhibitor include, but are not limited to, cantharidic acid and/or cantharidin. The term "tyrosine phosphatase inhibitor", as used here, relates to a compouns which targets, decreases or inhibits tyrosine phosphatase. Protein tyrosine phosphatases (PTPs) are relatively recent additions to the phosphatase family. They remove phosphate groups from phosphorylated tyrosine residues of proteins. PTPs display diverse structural features and play important roles in the regulation of cell proliferation, differentiation, cell adhesion and motility and cytoskeletal function. Examples of targets of a tyrosine phosphatase inhibitor include, but are not limited to, alkaline phosphatase (ALP), heparanase, PTPase, and/or prostatic acid phosphatase. Examples of a tyrosine phosphatase inhibitor include, but are not limited to, L-P-bromotetramisole oxalate; 2 (5H)-furanone,4-hydroxy-5-(hydroxymethyl)-3-(1-oxohexadecyl)-, (5R)-(9Cl); and benzylphosphonic acid.

The term "a PKC inhibitor", as used herein, relates to a compound which targets, decreases or inhibits PKC, as well as its isozymes. PKC, a ubiquitous, phospholipid-dependent enzyme, is involved in signal transduction associated with cell proliferation, differentiation and apoptosis. Examples of a target of a PKC inhibitor include, but are not limited to, MAPK and/or NF-kappaB. Examples of a PKC inhibitor include, but are not limited to, 1 H-pyrrolo-2,5-dione, 3-[1-[3-(dimethylamino)propyl]-1H-indol-3-yl]-4-(1H-indol-3-yl)-(9Cl); Bisindolylmaleimide IX; Sphingosine, which is known as 4-octadecene-1,3-diol, 2-amino-, (2S,3R,4E)-(9Cl); staurosporine, which is known as 9,13-epoxy-1H,9H-diindolo[1,2,3-gh:3',2',1'-Im]pyrrolo[3,4-j][1,7]benzodiazonin-1-one, 2,3,10,11,12,13-hexahydro-10-methoxy-9-methyl-11-(methylamino)-, (9S,10R,11R,13R)-(9Cl); tyrphostin 51; and Hypericin, which is also known as phenanthro[1,10,9,8-opqra]perylene-7,14-dione, 1,3,4,6,8,13-hexahydroxy-10,11-dimethyl-, stereoisomer (6Cl,7Cl,8Cl,9Cl).

The term "a PKC delta kinase inhibitor", as used herein, relates to a compound which targets, decreases or inhibits the delta isozymes of PKC. The delta isozyme is a conventional PKC isozymes and is $Ca^{2+}$-dependent. An example of a PKC delta kinase inhibitor includes, but is not limited to, Rottlerin, which is also known as 2-Propen-1-one, 1-[6-[(3-acetyl-2,4,6-trihydroxy-5-methylphenyl)methyl]-5,7-dihydroxy-2,2-dimethyl-2H-1-benzopyran-8-yl]-3-phenyl-, (2E)-(9Cl).

The term "a polyamine synthesis inhibitor", as used herein, relates to a compound which targets, decreases or inhibits polyamines spermidine. The polyamines spermidine and spermine are of vital importance for cell proliferation, although their precise mechanism of action is unclear. Tumor cells have an altered polyamine homeostasis reflected by increased activity of biosynthetic enzymes and elevated polyamine pools. Examples of a a polyamine synthesis inhibitor include, but are not limited to, DMFO, which is also known as (-)-2-difluoromethylornithin; N1, N12-diethylspermine 4HCl.

The term "a proteosome inhibitor", as used herein, relates to a compound which targets, decreases or inhibits proteosome. Examples of targets of a proteosome inhibitor include, but are not limited to, O(2)(-)-generating NADPH oxidase, NF-kappaB, and/or farnesyltransferase, geranylgeranyltransferase I. Examples of a proteosome inhibitor include, but are not limited to, aclacinomycin A; gliotoxin; PS-341; MLN 341; bortezomib; or Velcade.

The term "a PTP1B inhibitor", as used herein, relates to a compound which targets, decreases or inhibits PTP1 B, a protein tyrosine kinase inhibitor. An example of a PTP1 B inhibitor includes, but is not limited to, L-leucinamide, N-[4-(2-carboxyethenyl)benzoyl]glycyl-L-α-glutamyl-,(E)-(9Cl).

The term "a protein tyrosine kinase inhibitor", as used herein, relates to a compound which which targets, decreases or inhibits protein tyrosine kinases. Protein tyrosine kinases (PTKs) play a key role in the regulation of cell proliferation, differentiation, metabolism, migration and survival. They are classified as receptor PTKs and non-receptor PTKs. Receptor PTKs contain a single polypeptide chain with a transmembrane segment. The extracellular end of this segment contains a high affinity ligand-binding domain, while the cytoplasmic end comprises the catalytic core and the regulatory sequences. Examples of targets of a tyrosine kinase inhibitor include, but are not limited to, ERK1, ERK2, Bruton's tyrosine kinase (Btk), JAK2, ERK 1/2, PDGFR and/or FLT3. Examples of indirect targets include, but are not limited to, TNFα, NO, PGE2, IRAK, iNOS, ICAM-1 and/or E-selectin. Examples of a tyrosine kinase inhibitor include, but are not limited to, Tyrphostin AG 126; Tyrphostin Ag 1288; Tyrphostin Ag 1295; Geldanamycin; and Genistein.

Non-receptor tyrosine kinases include members of the SRC, Tec, JAK, Fes, Abl, FAK, Csk and Syk families. They are located in the cytoplasm, as well as in the nucleus. They exhibit distinct kinase regulation, substrate phosphorylation and function. Deregulation of these kinases has also been linked to several human diseases.

The term "a SRC family tyrosine kinase inhibitor", as used herein, relates to a compound which which targets, decreases or inhibits SRC. Examples of a SRC family tyrosine kinase inhibitor include, but are not limited to, PP1, which is also known as 1H-pyrazolo[3,4-d]pyrimidin-4-amine, 1-(1,1-dimethylethyl)-3-(1-naphthalenyl)-(9Cl); and PP2, which

is also known as 1H-Pyrazolo[3,4-d]pyrimidin-4-amine, 3-(4-chlorophenyl)-1-(1,1-dimethylethyl)-(9Cl).

The term "a Syk tyrosine kinase inhibitor", as used herein, relates to a compound which targets, decreases or inhibits Syk. Examples of targets for a Syk tyrosine kinase inhibitor include, but are not limited to, Syk, STAT3 and/or STAT5. An example of a Syk tyrosine kinase inhibitor includes, but is not limited to, Piceatannol, which is also known as 1,2-benzenediol, 4-[(1E)-2-(3,5-dihydroxyphenyl)ethenyl]-(9Cl).

The term "a Janus (JAK-2 and/or JAK-3) tyrosine kinase inhibitor", as used herein, relates to a compound which targets, decreases or inhibits janus tyrosine kinase. Janus tyrosine kinase inhibitor are shown anti-leukemic agents with anti-thrombotic, anti-allergic and immunosuppressive properties. Targets of a JAK-2 and/or JAK-3 tyrosine kinase inhibitor include, but are not limited to, JAK2, JAK3, STAT3. An indirect target of an JAK-2 and/or JAK-3 tyrosine kinase inhibitor includes, but is not limited to CDK2. Examples of a JAK-2 and/or JAK-3 tyrosine kinase inhibitor include, but are not limited to, Tyrphostin AG 490; and 2-naphthyl vinyl ketone.

The term "inhibitor of Ras oncogenic isoforms", as used herein, includes, but is not limited to H-Ras, K-Ras or N-Ras, as used herein, refers to compounds which target, decrease or inhibit the oncogenic activity of Ras, e.g., a farnesyl transferase inhibitor (FTI), e.g., L-744832, DK8G557 or R115777 (ZARNESTRA).

The term "a retinoid", as used herein, erfers to compounds that target, decrease or inhibit retinoid dependent receptors. Examples include, but are not limited to, Isotretinoin and Tretinoin.

The term "ribonucleotide reductase inhibitor", as used herein, includes, but is not limited to, pyrimidine or purine nucleoside analogs including, but not limited to, fludarabine and/or ara-C; 6-thioguanine; 5-FU; cladribine; 6-mercaptopurine, especially in combination with ara-C against ALL; and/or pentostatin. Ribonucleotide reductase inhibitors are especially hydroxyurea or 2-hydroxy-1H-isoindole-1,3-dione derivatives, such as PL-1, PL-2, PL-3, PL-4, PL-5, PL-6, PL-7 or PL-8. See Nandy et al., Acta Oncologica, Vol. 33, No. 8, pp. 953-961 (1994).

The term "a RNA polymerase II elongation inhibitor", as used herein, relates to a compound which targets, decreases or inhibits insulin-stimulated nuclear and cytosolic p70S6 kinase in CHO cells; targets, decreases or inhibits RNA polymerase II transcription, which may be dependent on casein kinase II; and targets, decreases or inhibits germinal vesicle breakdown in bovine oocytes An example of a RNA polymerase II elongation inhibitor includes, but is not limited to, 5,6-dichloro-1-beta-D-ribofuranosylbenzimidazole.

The term "S-adenosylmethionine decarboxylase inhibitors", as used herein, includes, but is not limited to, the compounds disclosed in U.S. Patent No. 5,461,076.

The term "a serine/threonine kinase inhibitor", as used herein, relates to a compound which inhibits serine/threonine kinases. An example of a target of a serine/threonine kinase inhibitor includes, but is not limited to, dsRNA-dependent protein kinase (PKR). Examples of indirect targets of a serine/threonine kinase inhibitor include, but are not limited to, MCP-1, NF-kappaB, elF2alpha, COX2, RANTES, IL8,CYP2A5, IGF-1, CYP2B1, CYP2B2, CYP2H1, ALAS-1, HIF-1, erythropoietin and/or CYP1A1. An example of a serine/theronin kinase inhibitor includes, but is not limited to, Sorafenib and 2-aminopurine, also known as 1 H-purin-2-amine(9Cl). Sorafenib is marketed as NEXAVAR.

The phrase "compound which targets, decreases or inhibits the activity/function of serine/theronine mTOR kinase", as used herein, includes, but is not limited to, compounds, proteins or antibodies which target/inhibit members of the mTOR kinase family, e.g., RAD, RAD001, CCI-779, ABT578, SAR543, rapamycin and derivatives/analogs thereof, AP23573 and AP23841 from Ariad, everolimus (CERTICAN) and sirolimus (RAPAMUNE), CCI-779 and ABT578. CERTICAN (everolimus, RAD) an investigational novel proliferation signal inhibitor that prevents proliferation of T-cells and vascular smooth muscle cells.

The term "somatostatin receptor antagonist", as used herein, includes, but is not limited to, agents which target, treat or inhibit the somatostatin receptor, such as octreoride and SOM230.

The term "a sterol biosynthesis inhibitor", as used herein, relates to a compound which inhibits the biosynthesis of sterols, such as cholesterol. Examples of targets for a sterol biosynthesis inhibitor include, but are not limited to, squalene epoxidase, and CYP2D6. An example of a sterol biosynthesis inhibitor includes, but is not limited to, terbinadine.

The term "telomerase inhibitor", as used herein, includes, but is not limited to, compounds which target, decrease or inhibit the activity of telomerase. Compounds which target, decrease or inhibit the activity of telomerase are especially compounds which inhibit the telomerase receptor, e.g., telomestatin.

The term "a topoisomerase inhibitor", includes a topoisomerase I inhibitor and a topoisomerase II inhibitor. Examples of a topoisomerase I inhibitor include, but are not limited to, topotecan, gimatecan also known as LBQ707, irinotecan, camptothecian and its analogues, 9-nitrocamptothecin and the macromolecular camptothecin conjugate PNU-166148 (compound A1 in WO 99/17804); 10-hydroxycamptothecin acetate salt; etoposide; idarubicin hydrochloride; irinotecan hydrochloride; teniposide; topotecan hydrochloride; doxorubicin; epirubicin hydrochloride; mitoxantrone hydrochloride; and daunorubicin hydrochloride. Irinotecan can be administered, e.g., in the form as it is marketed, e.g., under the trademark CAMPTOSAR. Topotecan can be administered, e.g., in the form as it is marketed, e.g., under the trademark HYCAMTIN. The term "topoisomerase II inhibitor", as used herein, includes, but is not limited to, the anthracyclines, such as doxorubicin, including liposomal formulation, e.g., CAELYX, daunorubicin, including liposomal formulation, e.g., DAUNOSOME, epirubicin, idarubicin and nemorubicin; the anthraquinones mitoxantrone and losoxantrone;

and the podophillotoxines etoposide and teniposide. Etoposide is marketed as ETOPOPHOS; teniposide as VM 26-BRISTOL; doxorubicin as ADRIBLASTIN or ADRIAMYCIN; epirubicin as FARMORUBICIN; idarubicin as ZAVEDOS; and mitoxantrone as NOVANTRON.

The phrase "tumor cell damaging approaches" refers to approaches, such as ionizing radiation. The term "ionizing radiation", referred to above and hereinafter, means ionizing radiation that occurs as either electromagnetic rays, such as X-rays and gamma rays; or particles, such as $\alpha$, $\beta$ and $\gamma$ particles. Ionizing radiation is provided in, but not limited to, radiation therapy and is known in the art. See Hellman, Cancer, 4th Edition, Vol. 1, Devita et al., Eds., pp. 248-275 (1993).

The phrase "a monoclonal antibody of VEGF or VEGFR", as used herein, includes but is not limited to, compounds disclosed in WO 98/35958, e.g., 1-(4-chloroanilino)-4-(4-pyridylmethyl)phthalazine or a pharmaceutically acceptable salt thereof, e.g., the succinate, or in WO 00/09495, WO 00/27820, WO 00/59509, WO 98/11223, WO 00/27819 and EP 0 769 947; those as described by Prewett et al., Cancer Res, Vol. 59, pp. 5209-5218 (1999); Yuan et al., Proc Natl Acad Sci USA, Vol. 93, pp. 14765-14770 (1996); Zhu et al., Cancer Res, Vol. 58, pp. 3209-3214 (1998); and Mordenti et al., Toxicol Pathol, Vol. 27, No. 1, pp. 14-21 (1999) in WO 00/37502 and WO 94/10202; ANGIOSTATIN, described by O'Reilly et al., Cell, Vol. 79, pp. 315-328 (1994); ENDOSTATIN, described by O'Reilly et al., Cell, Vol. 88, pp. 277-285 (1997); anthranilic acid amides; ZD4190; ZD6474; SU5416; SU6668; bevacizumab; or anti-VEGF antibodies or anti-VEGF receptor antibodies, e.g., rhuMAb and RHUFab; VEGF aptamer, e.g., Macugon; FLT-4 inhibitors; FLT-3 inhibitors; VEGFR-2 IgG1 antibody; Angiozyme (RPI 4610); and Avastan.

The term "VEGFR tyrosine kinase inhibitor", as used herein, relates to a compound which targets, decreases and/or inhibits the known angiogenic growth factors and cytokines implicated in the modulation of normal and pathological angiogenesis. The VEGF family (VEGF-A, VEGF-B, VEGF-C, VEGF-D) and their corresponding receptor tyrosine kinases [VEGFR-1 (Flt-1), VEGFR-2 (Flk-1, KDR), and VEGFR-3 (Flt-4)] play a paramount and indispensable role in regulating the multiple facets of the angiogenic and lymphangiogenic processes. An example of a VEGFR tyrosine kinase inhibitor includes, but is not limited to, 3-(4-dimethylaminobenzylidenyl)-2-indolinone.

The term "RANKL inhibitor", as used herein, relates to a compound that targets, decreases or inhibits RANK/RANKL pathway. RANK inhibitors prevent osteoclast-mediated bone loss in a range of conditions including osteoporosis, treatment-induced bone loss (bone loss due to glucocorticoid treatment and immunosuppression), rheumatoid arthritis, bone metastases and multiple myeloma. An example of a RANKL inhibitor includes, but is not limited to, denosumab.

In each case where citations of patent applications or scientific publications are given, in particular with regard to the respective compound claims and the final products of the working examples therein, the subject matter of the final products, the pharmaceutical preparations and the claims is hereby incorporated into the present application by reference to these publications. Comprised are likewise the corresponding stereoisomers, as well as the corresponding crystal modifications, e.g., solvates and polymorphs, which are disclosed therein. The compounds used as active ingredients in the combinations disclosed herein can be prepared and administered as described in the cited documents, respectively.

The structure of the active agents identified by code numbers, generic or trade names may be taken from the actual edition of the standard compendium "The Merck Index" or from databases, e.g., Patents International, e.g., IMS World Publications, or the publications mentioned above and below. The corresponding content thereof is hereby incorporated by reference.

It will be understood that references to the components (a) and (b) are meant to also include the pharmaceutically acceptable salts of any of the active substances. If active substances comprised by components (a) and/or (b) have, e.g., at least one basic center, they can form acid addition salts. Corresponding acid addition salts can also be formed having, if desired, an additionally present basic center. Active substances having an acid group, e.g., COOH, can form salts with bases. The active substances comprised in components (a) and/or (b) or a pharmaceutically acceptable salts thereof may also be used in form of a hydrate or include other solvents used for crystallization.

I. <u>The Combinations</u>

The present invention relates to a combination of:

(a) N-hydroxy-3-[4-[[[2-(2-methyl-1 H-indol-3-yl)-ethyl]-amino]methyl]phenyl]-2E-2-propenamide; and

(b) an pharmaceutically active agent.

In preferred embodiment, the present invention provides a combination comprising:

(a) N-hydroxy-3-[4-[[[2-(2-methyl-1 H-indol-3-yl)-ethyl]-amino]methyl]phenyl]-2E-2-propenamide; and

(b) one or more pharmaceutically active agents selected from the group consisting of an ACE inhibitor; an adenosine-kinase-inhibitor; an adjuvant; an adrenal cortex antagonist; AKT pathway inhibitor; an alkylating agent; an angiogenesis inhibitor; an angiostatic steroid; an anti-androgen; an anti-estrogen; an anti-hypercalcemia agent; an anti-leukemic compound; an anti-metabolite; an anti-proliferative antibody; an apoptosis inducer; an AT1 receptor antagonist; an aurora kinase inhibitor; an aromatase inhibitor; a biological response modifier; a bisphosphonate; a Bruton's Tyrosine Kinase (BTK) inhibitor; a calcineurin inhibitor; a CaM kinase II inhibitor; a CD45 tyrosine phosphatase inhibitor; a CDC25 phosphatase inhibitor; a CHK kinase inhibitor; a compound targeting/decreasing a protein or lipid kinase activity or a protein or lipid phosphatase activity, a further anti-angiogenic compound or a compound which induces cell differentiation processes; a controlling agent for regulating genistein, olomucine and/or tyrphostins; a cyclooxygenase inhibitor; a cRAF kinase inhibitor; a cyclin dependent kinase inhibitor; a cysteine protease inhibitor; a DNA intercalator; a DNA strand breaker; an E3 Ligase inhibitor; an EDG binder; an endocrine hormone; compounds targeting, decreasing or inhibiting the activity of the epidermal growth factor family; an EGFR, PDGFR tyrosine kinase inhibitor; a farnesyltransferase inhibitor; a Flk-1 kinase inhibitor; a compound which targets, decreases or inhibits the activity of Flt-3; a gonadorelin agonist; a Glycogen synthase kinase-3 (GSK3) inhibitor; a heparanase inhibitor; an agent used in the treatment of hematologic malignancies; a histone deacetylase (HDAC) inhibitor; a HSP90 inhibitor: an implant containing corticosteroids; a I-kappa B-alpha kinase inhibitor (IKK); an insulin receptor tyrosine kinase inhibitor; a c-Jun N-terminal kinase (JNK) kinase inhibitor; a microtubule binding agent; a MAP kinase inhibitor; a MDM2 inhibitor; a MEK inhibitor; a methionine aminopeptidase inhibitor; a matrix metalloproteinase (MMP) inhibitor; a monoclonal antibody; a NGFR tyrosine-kinase-inhibitor; a p38 MAP kinase inhibitor, including a SAPK2/p38 kinase inhibitor; a p56 tyrosine kinase inhibitor; a PDGFR tyrosine kinase inhibitor; a phosphatidylinositol 3-kinase inhibitor; a phosphatase inhibitor; photodynamic therapy; a platinum agent; a protein phosphatase inhibitor, including a PP1 and PP2 inhibitor and a tyrosine phosphatase inhibitor; a PKC inhibitor and a PKC delta kinase inhibitor; a polyamine synthesis inhibitor; a proteosome inhibitor; a PTP1 B inhibitor; a protein tyrosine kinase inhibitor including a SRC family tyrosine kinase inhibitor; a Syk tyrosine kinase inhibitor; and a JAK-2 and/or JAK-3 tyrosine kinase inhibitor; an inhibitor of Ras oncogenic isoforms; a retinoid; a ribonucleotide reductase inhibitor; a RNA polymerase II elongation inhibitor; an S-adenosylmethionine decarboxylase inhibitor; a serine/threonine kinase inhibitor; a compound which targets, decreases or inhibits the activity/function of serine/theronine mTOR kinase; a somatostatin receptor antagonist; a sterol biosynthesis inhibitor; a telomerase inhibitor; a topoisomerase inhibitor; tumor cell damaging approaches; a monoclonal antibody of VEGF or VEGFR; VEGFR tyrosine kinase inhibitor; and a RANKL inhibitor.

In another preferred embodiment, the present invention provides a combination comprising:

(a) N-hydroxy-3-[4-[[[2-(2-methyl-1 H-indol-3-yl)-ethyl]-amino]methyl]phenyl]-2E-2-propenamide; and

(b) one or more pharmaceutically active agents selected from the group consisting of CIBACEN; benazepril; enazepril; captopril; enalapril; fosinopril; lisinopril; moexipril; quinapril; ramipril; perindopril; trandolapril; 5-lodotubercidin; Leucovorin; Levamisole; Mitotane; Deguelin; Trciribine; Chlorambucil; cyclophosphamide; Dacarbazine; Lomustine; Procarbazine; Thiotepa; Melphalan; Temozolomide; Carmustine; Ifosfamide; Mitomycin; Altretamine; Busulfan; Machlorethamine hydrochloride; nitrosourea; Streptozocin; estramustine; Fumagillin; Shikonin; Tranilast; ursolic acid; suramin; thalidomide; lenalidomide; anecortave; triamcinolone; hydrocortisone; 11-$\alpha$-epihydrocotisol; cortexolone; 17$\alpha$-hydroxyprogesterone; corticosterone; desoxycorticosterone; testosterone; estrone; dexamethasone; Nilutamide; bicalutamide; Toremifene; Letrozole; Testolactone; Anastrozole; Bicalutamide; Flutamide; Tamoxifen Citrate; Exemestane; Fulestrant; tamoxifen; fulvestrant; raloxifene; raloxifene hydrochloride; gallium (III) nitrate hydrate; pamidronate disodium; Ara-C; hypoxanthine; 6-mercaptopurine (6-MP); fludarabine phosphate; Cytarabine; Fludarabine; Flexuridine; Fluorouracil; Capecitabine; Raltitrexed; Methotrexate; Cladribine; Gemcitabine; Gemcitabine hydrochloride; Thioguanine; Hydroxyurea; 5-azacytidine; decitabine; edatrexate; pemetrexed; trastuzumab; trastuzumab-DM1, erlotinib; Panitumumab, bevacizumab; rituximab; PRO64553; ethanol, 2-[[3-(2,3-dichlorophenoxy)propyl]amino]-(9Cl); gambogic acid; Embelin; Arsenic Trioxide; DIOVAN; Binucleine 2; atamestane; exemestane; formestane; aminoglutethimide; roglethimide; pyridoglutethimide; trilostane; testolactone; ketokonazole; vorozole; fadrozole; anastrozole; letrozole; lymphokine; interferon $\gamma$; etridonic; clodronic; tiludronic; pamidronic; alendronic; ibandronic; risedronic; zoledronic acid; terreic acid; Cypermethrin; Deltamethrin; Fenvalerate; Tyrphostin 8; 5-Isoquinolinesulfonic acid, 4-[(2S)-2-[(5-isoquinolinylsulfonyl)methylamino]-3-oxo-3-(4-phenyl-1-piperazinyl)propyl]phenyl ester (9Cl); benzenesulfonamide, N-[2-[[[3-(4-chlorophenyl)-2-propenyl]methyl]amino]methyl]phenyl]-N-(2-hydroxyethyl)-4-methoxy-(9Cl); Phosphonic acid, [[2-(4-bromophenoxy)-5-nitrophenyl]hydroxymethyl]-(9Cl); 1,4-naphthalenedione, 2,3-bis[(2-hydroyethyl)thio]-(9Cl); Debromohymenialdisine; 7H-pyrrolo [2,3-d]pyrimidine derivatives, including {6-[4-(4-ethyl-piperazine-1-ylmethyl)-phenyl]-7H-pyrrolo[2,3-d]pyrinidinpyrimidin-4-yl]-((R)-1-phenyl-ethyl)-amine; BAY 43-9006; (4-tert-butyl-phenyl)-94-pyridin-4-ylmethyl-isoquinolin-1-yl)-amine; imatinib; SU101; SU6668; GFB-111; 4-amino-5-phenyl-7-cyclobutyl-pyrrolo[2,3-d]pyrimidine derivatives;

PD180970; AG957; NSC 680410; PD173955; BMS354825; midostaurin; UCN-01; safingol; BAY 43-9006; Bryostatin 1; Perifosine; Ilmofosine; RO 318220; RO 320432; GO 6976; Isis 3521; LY333531/LY379196; PD184352; QAN697; imatinib mesylate (GLEEVEC); tyrphostin or pyrymidylaminobenzamide and derivatives thereof; Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556; AG957 and adaphostin (4-{[(2,5-dihydroxyphenyl) methyl]amino}-benzoic acid adamantyl ester, NSC 680410, adaphostin); trastuzumab (HERCEPTIN®); cetuximab; Iressa; OSI-774; CI-1033; EKB-569; Lapatinib; E1.1, E2.4, E2.5, E6.2, E6.4, E2.11, E6.3 or E7.6.3; RAD;RAD001; CCI-779; ABT578; SAR543; rapamycin; AP23573; AP23841; everolimus; sirolimus; phosphatase 1; phosphatase 2A; PTEN; okadaic acid; TNP-470; retinoic acid, α-, γ- or δ-tocopherol or α-, γ- or δ-tocotrienol; Daidzein; Iso-Olomoucine; Tyrphostin 1; 1H-indole-3-acetamide, 1-(4-chlorobenzoyl)-5-methoxy-2-methyl-N-(2-phenylethyl)-(9Cl); 5-alkyl substituted 2-arylaminophenylacetic acid; celecoxib; rofecoxib; etoricoxib; valdecoxib; 5-methyl-2-(2'-chloro-6'-fluoroanilino)phenyl acetic acid, lumiracoxib; 3-(3,5-dibromo-4-hydroxybenzylidene)-5-iodo-1,3-dihydroindol-2-one; benzamide, 3-(dimethylamino)-N-[3-[(4-hydroxybenzoyl)amino]-4-methylphenyl]-(9Cl); N9-Isopropyl-Olomoucine; Olomoucine; Purvalanol B; Roascovitine; Indirubin; Kenpaullone; purvalanol A; Indirubin-3'-monooxime; 4-morpholinecarboxamide,N-[(1S)-3-fluoro-2-oxo-1-(2-phenylethyl)propyl]amino]-2-oxo-1-(phenylmethyl)ethyl]-(9Cl); Plicamycin; Dactinomycin; Bleomycin; N-((3,3,3-trifluoro-2-trifluoromethyl)propionyl)sulfa nilamide; FTY720; Leuprolide; megestrol acetate; OSI-774, Cl-1033, EKB-569, Lapatinib, E1.1, E2.4, E2.5, E6.2, E6.4, E2.11, E6.3 or E7.6.3; erlotinib; gefitinib; Tyrphostin 23; Tyrphostin 25; Tyrphostin 47; Tyrphostin 51; Tyrphostin AG 825; 2-propenamide, 2-cyano-3-(3,4-dihydroxyphenyl)-N-phenyl-,(2E)-(9Cl); Tyrphostin Ag 1478; Lavendustin A; 3-pyridineacetonitrile, α-[(3,5-dichlorophenyl)methylene]-, (αZ)-(9Cl); Tyrphostin 46; α-hydroxyfarnesylphosphonic acid; butanoic acid, 2-[[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl)-,1-methylethyl ester, (2S)-(9cl); Manumycin A; 2-propenamide, 2-cyano-3-[4-hydroxy-3,5-bis(1-methylethyl)phenyl]-N-(3-phenylpropyl)-,(2E)-(9Cl); N-benzoyl-staurosporine; midostaurin; SU11248; MLN518; abarelix; goserelin; goserelin acetate; indirubin-3'-monooxime; PI-88; 5-(2,4-Dihydroxy-5-isopropyl-phenyl)-4-(4-morpholin-4-ylmethyl-phenyl)-isoxazole-3-carboxylic acid ethylamide; 1-b-D-arabinofuransylcytosine; bisulfan; N-hydroxy-3-[4-[[(2-hydroxyethyl)[2-(1H-indol-3-yl)ethyl]-amino]methyl]phenyl]-2E-2-propenamide; Suberoylanilide hydroxamic acid; [4-(2-amino-phenylcarbamoyl)-benzyl]-carbamic acid pyridine-3-ylmethyl ester and derivatives thereof; butyric acid; pyroxamide; trichostatin A; Oxamflatin; apicidin; Depsipeptide; depudecin; trapoxin; depudecin; HC Toxin; sodium phenylbutyrate; suberoyl bis-hydroxamic acid; Trichostatin A; 17-allylamino, 17-demethoxygeldanamycin (17AAG); geldanamycin,17-demethoxy-17-(2-propenylamino)-(9Cl); Geldanamycin; fluocinolone; dexamethasone; 2-propenenitrile, 3-[(4-methylphenyl)sulfonyl]-, (2E)-(9Cl); hydroxyl-2-naphthalenylmethylphosphonic acid; pyrazoleanthrone; epigallocatechin gallate; Vinblastine Sulfate; Vincristine Sulfate; Vindesine; Vinorelbine; Docetaxel; Paclitaxel; vinorelbine; discodermolides; cochicine; epothilone derivatives; epothilone B; Epotholine A; benzenesulfonamide, N-[2-[[[3-(4-chlorophenyl)-2-propenyl]methyl]amino]methyl]phenyl]-N-(2-hydroxyethyl)-4-methoxy-(9Cl); trans-4-iodo, 4'-boranyl-chalcone; butanedinitrile, bis[amino[2-aminophenyl)thio] methylene]-(9Cl); bengamide or a derivative thereof; Actinonin; epigallocatechin gallate; marimastat; prinomastat; metastat; BMS-279251; BAY 12-9566; TAA211; MMI270B; AAJ996; Panitumumab, bevacizumab; cetuximab; trastuzumab; Ibritumomab tiuxetan; tositumomab; iodine I 131; Tyrphostin AG 879; Phenol, 4-[4-(4-fluorophenyl)-5-(4-pyridinyl)-1H-imidazol-2-yl]-(9Cl); benzamide, 3-(dimethylamino)-N-[3-[(4-hydroxybenzoyl)amino]-4-methylphenyl]-(9Cl); damnacanthal; Tyrphostin 46; Tyrphostin AG 1296; Tyrphostin 9; 1,3-butadiene-1,1,3-tricarbonitrile,2-amino-4-(1H-indol-5-yl)-(9Cl); Wortmannin; Quercetin Dihydrate; cantharidic acid; cantharidin; L-leucinamide, N-[4-(2-carboxyethenyl)benzoyl]glycyl-L-α-glutamyl-,(E)-(9Cl); VISUDYNE; porfimer sodium; Carboplatin; Cisplatin; Oxaliplatin; cisplatinum; Satraplatin; such as ZD0473; cantharidic acid; cantharidin; L-P-bromotetramisole oxalate; 2(5H)-furanone,4-hydroxy-5-(hydroxymethyl)-3-(1-oxohexadecyl)-, (5R)-(9Cl); benzylphosphonic acid; 1 H-pyrrolo-2,5-dione,3-[1-[3-(dimethylamino)propyl]-1H-indol-3-yl]-4-(1H-indol-3-yl)-(9Cl); Bisindolylmaleimide IX; Sphingosine; staurosporine; tyrphostin 51; Hypericin; Rottlerin; DMFO; aclacinomycin A; gliotoxin; PS-341; MLN 341; bortezomib; Velcade; L-leucinamide, N-[4-(2-carboxyethenyl)benzoyl]glycyl-L-α-glutamyl-,(E)-(9Cl); Tyrphostin AG 126; Tyrphostin Ag 1288; Tyrphostin Ag 1295; Geldanamycin; Genistein; PP1; PP2; 1,2-Benzenediol, 4-[(1E)-2-(3,5-dihydroxyphenyl)ethenyl]-(9Cl); Tyrphostin AG 490; 2-naphthyl vinyl ketone; L-744832; DK8G557; R115777; Isotretinoin; Tretinoin; fludarabine; ara-C; 6-thioguanine; 5-FU; cladribine; 6-mercaptopurine; pentostatin; 5,6-dichloro-1-β-D-ribofuranosylbenzimidazole; Sorafenib, 2-aminopurine; CCI-779; ABT578; SAR543; rapamycin and derivatives thereof; AP23573; AP23841; sirolimus; CCI-779; ABT578; octreotide; SOM230; squalene epoxidase; CYP2D6; terbinadine; telomestatin; topotecan; gimatecan; irinotecan; camptothecin; 9-nitrocamptothecin; PNU-166148; 10-hydroxycamptothecin acetate salt; etoposide; idarubicin hydrochloride; irinotecan hydrochloride; teniposide; topotecan hydrochloride; doxorubicin; epirubicin hydrochloride; mitoxantrone hydrochloride; daunorubicin hydrochloride; doxorubicin; epirubicin; idarubicin; nemorubicin; losoxantrone; teniposide; etoposide; mitoxantrone; 1-(4-chloroanilino)-4-(4-pyridylmethyl)phthalazine or a pharmaceutically acceptable salt thereof; ZD4190; ZD6474; SU5416; SU6668; Panitumumab, bevacizumab; rhuMAb; RHUFab; Macugon; Angiozyme Avastan; 3-(4-dimethylaminobenzylidenyl)-2-indolinone; denosumab.

Any of the combination of components (a) and (b), the method of treating a warm-blooded animal comprising administering these two components, a pharmaceutical composition comprising these two components for simultaneous, separate or sequential use, the use of the combination for the delay of progression or the treatment of a proliferative disease or for the manufacture of a pharmaceutical preparation for these purposes or a commercial product comprising such a combination of components (a) and (b), all as mentioned or defined above, will be referred to subsequently also as COMBINATION OF THE INVENTION (so that this term refers to each of these embodiments which thus can replace this term where appropriate).

Simultaneous administration may, e.g., take place in the form of one fixed combination with two or more active ingredients, or by simultaneously administering two or more active ingredients that are formulated independently. Sequential use (administration) preferably means administration of one (or more) components of a combination at one time point, other components at a different time point, that is, in a chronically staggered manner, preferably such that the combination shows more efficiency than the single compounds administered independently (especially showing synergism). Separate use (administration) preferably means administration of the components of the combination independently of each other at different time points, preferably meaning that the components (a) and (b) are administered such that no overlap of measurable blood levels of both compounds are present in an overlapping manner (at the same time).

Also combinations of two or more of sequential, separate and simultaneous administration are possible, preferably such that the combination component-drugs show a joint therapeutic effect that exceeds the effect found when the combination component-drugs are used independently at time intervals so large that no mutual effect on their therapeutic efficiency can be found, a synergistic effect being especially preferred.

The term "delay of progression", as used herein, means administration of the combination to patients being in a pre-stage or in an early phase, of the first manifestation or a relapse of the disease to be treated, in which patients, e.g., a pre-form of the corresponding disease is diagnosed or which patients are in a condition, e.g., during a medical treatment or a condition resulting from an accident, under which it is likely that a corresponding disease will develop.

"Jointly therapeutically active" or "joint therapeutic effect" means that the compounds may be given separately (in a chronically staggered manner, especially a sequence-specific manner) in such time intervals that they preferably, in the warm-blooded animal, especially human, to be treated, still show a (preferably synergistic) interaction (joint therapeutic effect). Whether this is the case, can inter alia be determined by following the blood levels, showing that both compounds are present in the blood of the human to be treated at least during certain time intervals.

"Pharmaceutically effective" preferably relates to an amount that is therapeutically or in a broader sense also prophylactically effective against the progression of a proliferative disease.

The term "a commercial package" or "a product", as used herein, defines especially a "kit of parts" in the sense that the components (a) and (b), as defined above, can be dosed independently or by use of different fixed combinations with distinguished amounts of the components (a) and (b), i.e., simultaneously or at different time points. Moreover, these terms comprise a commercial package comprising (especially combining) as active ingredients components (a) and (b), together with instructions for simultaneous, sequential (chronically staggered, in time-specific sequence, preferentially) or (less preferably) separate use thereof in the delay of progression or treatment of a proliferative disease. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b) (as can be determined according to standard methods. The ratio of the total amounts of the combination partner (a) to the combination partner (b) to be administered in the combined preparation can be varied, e.g., in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a more than additive effect, which hence could be achieved with lower doses of each of the combined drugs, respectively, than tolerable in the case of treatment with the individual drugs only without combination, producing additional advantageous effects, e.g., less side effects or a combined therapeutic effect in a non-effective dosage of one or both of the combination partners (components) (a) and (b), and very preferably a strong synergism of the combination partners (a) and (b).

Both in the case of the use of the combination of components (a) and (b) and of the commercial package, any combination of simultaneous, sequential and separate use is also possible, meaning that the components (a) and (b) may be administered at one time point simultaneously, followed by administration of only one component with lower host toxicity either chronically, e.g., more than 3-4 weeks of daily dosing, at a later time point and subsequently the other component or the combination of both components at a still later time point (in subsequent drug combination treatment courses for an optimal anti-tumor effect) or the like.

The COMBINATION OF THE INVENTION can also be applied in combination with other treatments, e.g.,

surgical intervention, hyperthermia and/or irradiation therapy.

The pharmaceutical compositions according to the present invention can be prepared by conventional means and are those suitable for enteral, such as oral or rectal, and parenteral administration to mammals including man, comprising a therapeutically effective amount of a VEGF inhibitor and at least one pharmaceutically active agent alone or in combination with one or more pharmaceutically acceptable carriers, especially those suitable for enteral or parenteral application.

The pharmaceutical compositions comprise from about 0.00002% to about 100%, especially, e.g., in the case of infusion dilutions that are ready for use) of 0.0001-0.02%, or, e.g., in case of injection or infusion concentrates or especially parenteral formulations, from about 0.1% to about 95%, preferably from about 1% to about 90%, more preferably from about 20% to about 60%.

The effective dosage of each of the combination partners employed in a formulation of the present invention may vary depending on the particular compound or pharmaceutical compositions employed, the mode of administration, the condition being treated and the severity of the condition being treated. A physician, clinician or veterinarian of ordinary skill can readily determine the effective amount of each of the active ingredients necessary to prevent, treat or inhibit the progress of the condition.

Pharmaceutical preparations for the combination therapy for enteral or parenteral administration are, e.g., those in unit dosage forms, such as sugar-coated tablets, capsules or suppositories, and furthermore ampoules. If not indicated otherwise, these formulations are prepared by conventional means, e.g., by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units. One of skill in the art has the ability to determine appropriate pharmaceutically effective amounts of the combination components.

Preferably, the compounds or the pharmaceutically acceptable salts thereof, are administered as an oral pharmaceutical formulation in the form of a tablet, capsule or syrup; or as parenteral injections if appropriate.

In preparing compositions for oral administration, any pharmaceutically acceptable media may be employed such as water, glycols, oils, alcohols, flavoring agents, preservatives, coloring agents. Pharmaceutically acceptable carriers include starches, sugars, microcrystalline celluloses, diluents, granulating agents, lubricants, binders, disintegrating agents.

Solutions of the active ingredient, and also suspensions, and especially isotonic aqueous solutions or suspensions, are useful for parenteral administration of the active ingredient, it being possible, e.g., in the case of lyophilized compositions that comprise the active ingredient alone or together with a pharmaceutically acceptable carrier, e.g., mannitol, for such solutions or suspensions to be produced prior to use. The pharmaceutical compositions may be sterilized and/or may comprise excipients, e.g., preservatives, stabilizers, wetting and/or emulsifying agents, solubilizers, salts for regulating the osmotic pressure and/or buffers, and are prepared in a manner known *per se,* e.g., by means of conventional dissolving or lyophilizing processes. The solutions or suspensions may comprise viscosity-increasing substances, such as sodium carboxymethylcellulose, carboxymethylcellulose, dextran, polyvinylpyrrolidone or gelatin. Suspensions in oil comprise as the oil component the vegetable, synthetic or semi-synthetic oils customary for injection purposes.

The isotonic agent may be selected from any of those known in the art, e.g. mannitol, dextrose, glucose and sodium chloride. The infusion formulation may be diluted with the aqueous medium. The amount of aqueous medium employed as a diluent is chosen according to the desired concentration of active ingredient in the infusion solution. Infusion solutions may contain other excipients commonly employed in formulations to be administered intravenously, such as antioxidants.

The present invention further relates to "a combined preparation", which, as used herein, defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. The ratio of the total amounts of the combination partner (a) to the combination partner (b) to be administered in the combined preparation can be varied, e.g., in order to cope with the needs of a patient sub-population to be treated or the needs of the single patient based on the severity of any side effects that the patient experiences.

The present invention especially relates to a combined preparation which comprises:

(a) one or more unit dosage forms of N-hydroxy-3-[4-[[[2-(2-methyl-1H-indol-3-yl)-ethyl]-amino]methyl]phenyl]-2E-2-propenamide; and

(b) one or more unit dosage forms of an pharmaceutically active agent.

The compositions of the present invention are useful for treating proliferative diseases or diseases that are associated with or triggered by persistent angiogenesis.

A proliferative disease is mainly a tumor disease (or cancer) (and/or any metastases). The inventive compositions are particularly useful for treating a tumor which is a breast cancer, lung cancer, including non-small cell lung carcinoma, renal cancer, colon cancer, myelodysplastic syndrome, genitourinary cancer, gastrointestinal cancer, epidermoid cancer, melanoma, glioma, ovarian cancer, pancreatic cancer, lymphoma, myeloma, colorectal cancer, neuroblastoma, head and/or neck cancer or bladder cancer, or in a broader sense brain or gastric cancer.

In a broader sense of the invention, a proliferative disease may furthermore be a hyperproliferative condition, such as leukemias, hyperplasias, fibrosis (especially pulmonary, but also other types of fibrosis, such as renal fibrosis), angiogenesis, psoriasis, atherosclerosis and smooth muscle proliferation in the blood vessels, such as stenosis or restenosis following angioplasty.

Where a tumor, a tumor disease, a carcinoma or a cancer are mentioned, also metastasis in the original organ or tissue and/or in any other location are implied alternatively or in addition, whatever the location of the tumor and/or metastasis.

The compositions are selectively toxic or more toxic to rapidly proliferating cells than to normal cells, particularly in human cancer cells, e.g., cancerous tumors, the compound has significant anti-proliferative effects and promotes differentiation, e.g., cell cycle arrest and apoptosis.

**Brief description of the drawings:**

Fig. 1 MV4-11 cells were treated with the indicated doses of AUY922 and/or LBH589 for 48 hours. Following this, the percentages of annexin V-positive apoptotic cells were determined by flow cytometry. Values represent the mean $\pm$ S.E.M. of three experiments.

Fig. 2 Analysis of dose effect relationship for AUY922 and LBH589 for the apoptotic effects after 48 hours of exposure was done according to the median effect of Chou and Talalay. Then the combination index values were calculated. CI values less than 1.0 reflect synergism of the two agents.

The invention is illustrated by the following Examples.

**Example 1**

Methods

*Cell Culture*

Cell lines derived from human tumors e.g. breast (BT474, SKBR3, MDA-MB-453, MCF7), gastric (N-87), prostate (CWR22Rv1) Lung (A549), melanoma (SKMEL28), Ovarian (SKOV3) were cultured according to established conditions. Cells were generally maintained in artificial media, such as Dubelco Modified Eagle Medium (DMEM) or RPMI and supplemented with various levels up to15% fetal bovine serum. The antibiotics penicillin 100 units/mL) and streptomycin (100 μg/mL) were added to prevent bacterial contamination and maintained at 37°C and 5% $CO_2$ environment in a sterile incubator.

*Monolayer Growth Inhibition Assay*

Three methods of cell growth inhibition assays were generally used. They are: 1) the Cell Titer Glow Assay, 2) the Alamar Blue Fluorometric Assay and 3) the MTT Assay Cell Proliferation Assay. $IC_{25}$, $IC_{50}$, $IC_{75}$ or $IC_{90}$ the concentration of compound that inhibits 25%, 50%, 75% or 90% of the cells after incubation for a specified number of hours were used as a measure of antiproliferative potency.

The CellTiter-Glo® Luminescent Cell Viability Assay is a homogeneous method of determining the number of viable cells in culture based on quantitation of the ATP present, which signals the presence of metabolically active cells. The CellTiter-Glo® Assay generates a "glow-type" luminescent signal, produced by the luciferase reaction. The luminescent signal is proportional to the amount of ATP present, which is directly proportional to the number of cells present in culture.

AlamarBlue™ detects cell viability by utilizing a blue and nonfluorescent dye resazurin, which is converted to a pink and fluorescent dye resorufin in response to chemical reduction of growth medium resulting from cell growth.

Reduction related to growth causes the REDOX indicator to change from the oxidized (nonfluorescent, blue) form to the reduced (fluorescent, red) form. The fluorescent and colorimetric signal generated from the assay is proportional to the number of living cells in the sample.

The MTT is a colorimetric assay to determine the cell proliferation rate. The yellow tetrazolium MTT (3-(4, 5-dimethylthiazolyl-2)-2,5-diphenyltetrazolium bromide) is reduced by metabolically active cells, in part by the action of dehydrogenase enzymes, to generate reducing equivalents, such as NADH and NADPH. The resulting intracellular purple formazan can be solubilized and quantified by spectrophotometric means. The signals produced is directly proportional to the cell numbers. Describing the MTT assay in detail, experiments were done using 6-point or 9-point drug titrations in multi-well tissue culture dishes, with outer rows left empty. Cells were suspended in complete media at densities of between $10^3$ and $10^4$ cell/mL, respectively, and added per well. The appropriate medium (200 μL) was then added. Twenty-four hours later, 10 μL of MTS solution [5], were added to one plates to determine the activity at the time of compound addition (To). This plate was incubated at 37°C for 4 hours and the optical density was measured on a Molecular Devices Thermomax at 490 nm using the Softmax program. The To plate served as a reference for initial activity at the beginning of the experiment.

Compound addition began 24 hours after seeding, the same time as the To determination. Serial dilutions at 4-fold, 2-fold, 1-fold, 0.5-fold, 0.25-fold and 0.125-fold of previously determined $IC_{50}$ values of each compound were made in a 96-deep well plate with the highest concentrations on the edge of the plate. Each of the 6 dilutions were added in triplicate and complete medium was added to the empty outer rows without cells. The compounds were added to the plates singly or in combination with N-hydroxy-3-[4-[[[2-(2-methyl-1 H-indol-3-yl)-ethyl]-amino]methyl]phenyl]-2E-2-propenamide. The plates were incubated at 37°C for 72 hours from seeding. The MTS solution was added (as for the To plate) and read 4 hours later. In order to analyze the data, the average value of media alone (background) was subtracted from each experimental well and the triplicate values were averaged for each compound dilution. The following formulas were used to calculate percent growth.

$$\text{If } X > T_0,\ \%\ \text{Growth} = 100 \times ((X-T_0)/(GC - T_0))$$

$$\text{If } X < T_0,\ \%\ \text{Growth} = 100 \times (X-T_0)/T_0)$$

To = average value of To minus background

GC = average value of untreated cells (in triplicate) minus background

X = average value of compound treated cells (in triplicate) minus background

The "% Growth" was plotted against compound concentration and used to calculate $IC_{50}$s employing the user-defined spline function in Microsoft Excel. This function uses linear regression between data points to predict the concentration of compounds at 50% inhibition. $IC_{50}$s were used to determine the dose range for each compound and the resultant combinations.

*Combination Index (CI)*

To determine whether combination of N-hydroxy-3-[4-[[[2-(2-methyl-1H-indol-3-yl)-ethyl]-amino]methyl]phenyl]-2E-2-propenamide with other compounds had an additive effect, synergistic effect or an antagonistic effect together, a parameter known as a combination index was determined for the antiproliferative activity elicited by each combination. CI was determined by the isobologram equation CI = $(D)_1/(D_x)_1 + (D)_2/(D_x)_2$ Drug 1 $(D)_1$ and drug 2 $(D)_2$ in combination inhibit X% and $(D_x)_1$ and $(D_x)_2$ are the doses of drug 1 and drug 2 alone that also inhibits X%. For each compound we used the % growth values at each dose as determned in the MTS assay. CI values that less than 1 shows synergism, CI values equal to1 shows additivity and CI values greater than 1 indicate antagonism. CIs were generally determined at $IC_{50}$, however, in other cases determined at $IC_{25}$, $IC_{75}$ and $IC_{90}$ as well.

Table 1. Combination Indicies of combining N-hydroxy-3-[4-[[[2-(2-methyl-1H-indol-3-yl)-ethyl]-amino]methyl] phenyl]-2E-2-propenamide (also known as LBH589) with other anticancer agents in clinical or experimental use, using the the Alamar Blue Fluorometric assay. 2-[5-Chloro-2-(2-methoxy-4-morpholin-4-yl-phenylamino)-pyrimidin-4-ylamino]-N-methyl-benzamide is also known as TAE226. 7-Hydroxy-8,8,10,11,12,16-hexamethyl-3-[1-methyl-2-(2-methylthio-thiazol-4-yl)-vinyl]-4,17-dioxa-bicyclo[14.1.0]heptadecane-5,9-dione is known as ABJ879.

**Table 1**

| First Compound | Combination Compound | Cell Type | Tumor Type | CI ($IC_{25}$) | CI ($IC_{50}$) | CI ($IC_{75}$) | Combination Effect |
|---|---|---|---|---|---|---|---|
| LBH589 | Velcade | MIA PaCa-2 | Pancreatic | 0.03 | 0.22 | 1.14 | Synergistic |
| LBH589 | AEE788 | PANC-1 | Pancreatic | 0.08 | 0.33 | 0.54 | Synergistic |
| LBH589 | Paclitaxel | MIA PaCa-2 | Pancreatic | 0.14 | 0.30 | 0.49 | Synergistic |
| LBH589 | LBQ707 | A549 | Lung | 0.11 | 0.36 | 3.25 | Synergistic |
| LBH589 | AEE788 | A549 | Lung | 0.18 | 0.39 | 1.42 | Synergistic |
| LBH589 | ABJ879 | MIA PaCa-2 | Pancreatic | NA | 0.34 | 0.25 | Synergistic |
| LBH589 | AEW541 | RPMI8226 | Myeloma | 0.27 | 0.41 | 0.50 | Synergistic |
| LBH589 | LBQ707 | SW620 | Colon | 0.27 | 0.42 | 1.49 | Synergistic |
| LBH589 | ABJ879 | U266B1 | Lymphoma | 0.08 | NA | NA | Synergistic |
| LBH589 | Velcade | U266B1 | Lymphoma | 0.09 | NA | NA | Synergistic |
| LBH589 | AEW541 | U266B1 | Lymphoma | 0.11 | NA | NA | Synergistic |
| LBH589 | LBQ707 | U266B1 | Lymphoma | 0.12 | NA | NA | Synergistic |
| LBH589 | LBQ707 | RPMI8226 | Myeloma | NA | NA | 0.13 | Synergistic |
| LBH589 | ABJ879 | RPMI8226 | Myeloma | NA | NA | 0.15 | Synergistic |
| LBH589 | Gemcitabine | MIA PaCa-2 | Pancreatic | 0.16 | 0.47 | NA | Synergistic |
| LBH589 | Gemcitabine | U266B1 | Lymphoma | 0.16 | NA | NA | Synergistic |
| LBH589 | Paclitaxel | U266B1 | Lymphoma | 0.19 | NA | NA | Synergistic |
| LBH589 | PKC412 | SW620 | Colon | 0.20 | 0.46 | 1.40 | Synergistic |
| LBH589 | 5FU | MIA PaCa-2 | Pancreatic | 0.21 | 0.47 | 0.92 | Synergistic |
| LBH589 | ABJ879 | HeLa | Ovarian | NA | 0.27 | NA | Synergistic |
| LBH589 | Velcade | RPMI8226 | Myeloma | 0.27 | 0.48 | 0.51 | Synergistic |
| LBH589 | Paclitaxel | RPMI8226 | Myeloma | 0.27 | 0.51 | 0.58 | Synergistic |
| LBH589 | EPO906 | A549 | lung | 2.00 | 1.79 | 0.30 | Synergistic |
| LBH589 | PKC412 | MIA PaCa-2 | Pancreatic | 0.30 | 0.57 | 1.00 | Synergistic |
| LBH589 | PKC412 | HeLa | Ovarian | 0.32 | 0.48 | NA | Synergistic |
| LBH589 | LBQ707 | 786-O | Renal | 0.92 | 0.71 | 0.33 | Synergistic |
| LBH589 | AEE788 | MIA PaCa-2 | Pancreatic | 0.34 | 0.57 | 1.32 | Synergistic |
| LBH589 | EPO906 | U266B1 | Lymphoma | 0.34 | NA | NA | Synergistic |
| LBH589 | ABJ879 | HeLa | Ovarian | 0.34 | 0.65 | 1.08 | Synergistic |
| LBH589 | RAD001 | A549 | lung | 1.75 | 0.34 | NA | Synergistic |
| LBH589 | ABJ879 | PANC-1 | Pancreatic | NA | 0.35 | 0.88 | Synergistic |
| LBH589 | AEW541 | HeLa | | 0.37 | 0.49 | NA | Synergistic |
| LBH589 | PTK787 | MIA PaCa-2 | Pancreatic | 0.37 | 0.75 | 1.81 | Synergistic |
| LBH589 | PTK787 | U266B1 | Lymphoma | 0.37 | NA | NA | Synergistic |
| LBH589 | AAE581 | RPMI8226 | Myeloma | 0.38 | 0.56 | 0.59 | Synergistic |
| LBH589 | LBQ707 | HeLa | Ovarian | 0.49 | 0.39 | NA | Synergistic |
| LBH589 | Gemcitabine | 786-O | Renal | 1.08 | 0.87 | 0.41 | Synergistic |

(continued)

| First Compound | Combination Compound | Cell Type | Tumor Type | CI ($IC_{25}$) | CI ($IC_{50}$) | CI ($IC_{75}$) | Combination Effect |
|---|---|---|---|---|---|---|---|
| LBH589 | EPO906 | SW620 | Colo | 0.46 | 0.42 | 1.20 | Synergistic |
| LBH589 | 5FU | RPMI8226 | Myeloma | 0.44 | 0.56 | NA | Synergistic |
| LBH589 | AEW541 | MIA PaCa-2 | Pancreatic | 0.50 | 0.80 | 0.93 | Synergistic |
| LBH589 | LBQ707 | SKOV3 | | 0.92 | 0.64 | NA | Synergistic |
| LBH589 | TAE226 | HeLa | | NA | NA | NA | Synergistic |
| LBH589 | TAE226 | MIA PaCa-2 | | 0.54 | 0.71 | 0.80 | Synergistic |
| LBH589 | AEW541 | SW620 | | 0.51 | 0.68 | | Synergistic |

Table 2. Combination Indicies of combining N-hydroxy-3-[4-[[[2-(2-methyl-1H-indol-3-yl)-ethyl]-amino]methyl] phenyl]-2E-2-propenamide (also known as LBH589) with other anticancer agents in clinical or experimental use, using the MTT assay method

**Table 2**

| First Compound | Combination Compound(s) | CellType | Tumor Type | CI | Combination Effect |
|---|---|---|---|---|---|
| LBH589 | Idarubicin Hydrochloride | SKMEL28 | Melanoma | 0.56 | Synergistic |
| LBH589 | Staurosporine | SKMEL28 | Melanoma | 0.53 | Synergistic |
| LBH589 | Wortmannin | SKOV3 | Ovarian | 0.44 | Synergistic |
| LBH589 | Mitoxantrone Hydrochloride | A549 | Lung | 0.43 | Synergistic |
| LBH589 | Mitoxantrone Hydrochloride | SKOV3 | Ovarian | 0.43 | Synergistic |
| LBH589 | 2,3-DCPE HCL | SKMEL28 | Melanoma | 0.5 | Synergistic |
| LBH589 | Mitoxantrone Hydrochloride + Prednisone | SKMEL28 | Melanoma | 0.55 | Synergistic |
| LBH589 | Teniposide | SKOV3 | Ovarian | 0.41 | Synergistic |
| LBH589 | Mitoxantrone Hydrochloride + Prednisone | SKOV3 | Ovarian | 0.36 | Synergistic |
| LBH589 | 10-Hydroxycamptothecin Acetate Salt | SKMEL28 | Melanoma | 0.54 | Synergistic |
| LBH589 | Floxuridine | A549 | Lung | 0.9 | Synergistic |
| LBH589 | Mitomycin | SKOV3 | Ovarian | 0.51 | Synergistic |
| LBH589 | Mitoxantrone Hydrochloride + Prednisone | A549 | Lung | 0.53 | Synergistic |
| LBH589 | Mitoxantrone Hydrochloride | SKMEL28 | Melanoma | 0.57 | Synergistic |
| LBH589 | Cisplatin + Doxorubicin Hcl | SKMEL28 | Melanoma | 0.55 | Synergistic |
| LBH589 | Cisplatin + Doxorubicin Hcl | SKOV3 | Ovarian | 0.4 | Synergistic |
| LBH589 | Teniposide | A549 | Lung | 0.48 | Synergistic |
| LBH589 | Fluorouracil + Mitomycin | SKMEL28 | Melanoma | 0.75 | Synergistic |
| LBH589 | Idarubicin Hydrochloride | A549 | Lung | 0.53 | Synergistic |
| LBH589 | Idarubicin Hydrochloride | SKOV3 | Ovarian | 0.56 | Synergistic |
| LBH589 | Tyrphostin 9 | A549 | Lung | 0.51 | Synergistic |
| LBH589 | Fluorouracil + Mitomycin | SKOV3 | Ovarian | 0.57 | Synergistic |

(continued)

| First Compound | Combination Compound(s) | CellType | Tumor Type | CI | Combination Effect |
|---|---|---|---|---|---|
| LBH589 | NVP100 | SKOV3 | Ovarian | 0.4 | Synergistic |
| LBH589 | Epirubicin Hydrochloride | SKOV3 | Ovarian | 0.52 | Synergistic |
| LBH589 | Etoposide | A549 | Lung | 0.52 | Synergistic |
| LBH589 | Wortmannin | SKMEL28 | Melanoma | 0.74 | Synergistic |
| LBH589 | Cisplatin + Doxorubicin Hcl | A549 | Lung | 0.51 | Synergistic |
| LBH589 | Daunorubicin Hydrochloride + Cytarabine | SKOV3 | Ovarian | 0.3 | Synergistic |
| LBH589 | 10-Hydroxycamptothecin Acetate Salt | A549 | Lung | 0.4 | Synergistic |
| LBH589 | Thioguanine | SKMEL28 | Melanoma | 0.43 | Synergistic |
| LBH589 | Vincristine Sulfate | SKOV3 | Ovarian | 0.58 | Synergistic |
| LBH589 | Ketoconazole + Doxorubicin Hcl | SKOV3 | Ovarian | 0.5 | Synergistic |
| LBH589 | Etoposide + Estramustine Phosphate Sodium | SKOV3 | Ovarian | 0.51 | Synergistic |
| LBH589 | Paclitaxel | SKOV3 | Ovarian | 0.57 | Synergistic |
| LBH589 | 6-mercaptopurine monohydrate | SKOV3 | Ovarian | 0.52 | Synergistic |
| LBH589 | HC Toxin | SKMEL28 | Melanoma | 0.53 | Synergistic |
| LBH589 | Vindesine Sulfate | SKMEL28 | Melanoma | 0.44 | Synergistic |
| LBH589 | LM-4108 | A549 | Lung | 0.51 | Synergistic |
| LBH589 | HC Toxin | A549 | Lung | 0.59 | Synergistic |
| LBH589 | Procarbazine Hydrochloride | SKOV3 | Ovarian | 0.51 | Synergistic |
| LBH589 | Hydroxyurea | SKOV3 | Ovarian | 0.5 | Synergistic |
| LBH589 | Iso-Olomoucine | SKOV3 | Ovarian | 0.5 | Synergistic |
| LBH589 | Indirubin-3'-Monooxime | SKOV3 | Ovarian | 0.53 | Synergistic |
| LBH589 | Fluorouracil | SKOV3 | Ovarian | 0.57 | Synergistic |
| LBH589 | Piceatannol | SKOV3 | Ovarian | 0.43 | Synergistic |
| LBH589 | N1 N12-diethylspermine 4HCL | SKOV3 | Ovarian | 0.46 | Synergistic |
| LBH589 | L-744832 | SKOV3 | Ovarian | 0.45 | Synergistic |
| LBH589 | Indirubin-3'-Monooxime | SKOV3 | Ovarian | 0.53 | Synergistic |
| LBH589 | Fluorouracil | SKOV3 | Ovarian | 0.57 | Synergistic |
| LBH589 | Piceatannol | SKOV3 | Ovarian | 0.43 | Synergistic |
| LBH589 | N1 N12-diethylspermine 4HCL | SKOV3 | Ovarian | 0.46 | Synergistic |

Table 3. Combination sffects of combining N-hydroxy-3-[4-[[[2-(2-methyl-1H-indol-3-yl)-ethyl]-amino]methyl] phenyl]-2E-2-propenamide (also known as LBH589) with Lapatinib (Her2/neu and EGFR inhibitor), AEE788 Her2/neu and EGFR inhibitor), or Gefitinib (EGFR inhibitor) in breast cancer cell lines expressing Her2/neu, Estrogen receptor (ER) or both. These studies used the Cell titer Glow Assay.

Table 3

| Cell Lines | Genotypes | Combination (CI Values at $EC_{50}$) | | |
|---|---|---|---|---|
| | | LBH589 + Lapatinib | LBH589 + AEE788 | LBH589 + Gefitinib |
| BT474 | ER+/Her2+ | Synergy | Synergy | Additive |
| SK-BR-3 | ER-/Her2+ | Synergy | Synergy | Slight synergy |
| MDA-MB-453 | ER-/Her2+ | Synergy | Synergy | Additive |
| N87 | ER-/Her2+ | Synergy | Synergy | Additive |
| MCF7 | ER+/Her2- | Additive | Additive | Additive |

The effect of combined treatment of NVP-LBH589 with a EGFR/Her2 or EGFR inhibitor on breast cancer cell proliferation

For compound combination studies, cells were treated with both of serially diluted compounds. Viable cells were measured on day 3 of the treatment using the CellTiter-Glo assay. Combination Index value at 50% of growth inhibition compared to vehicle control ($CI_{50}$) was calculated as following:

$$CI_{50} = (C)_1/(Cm)_1 + (C)_2/(Cm)_2$$

$(C)_1 = EC_{50}$ of compound 1 in combination
$(Cm)_1 = EC_{50}$ of compound 1 alone
$(C)_2 = EC_{50}$ of compound 2 in combination
$(Cm)_2 = EC_{50}$ of compound 2 alone

The combination effect of NVP-LBH589 with either a dual EGFR/HER2 inhibitor lapatinib or a EFGR inhibitor gefitinib was measured by the value of combination index (CI). CI value of equal to one indicates additivity of the two compounds in combination. CI value of less than one indicates synergism, whereas CI value of greater than one suggests antagonism. As presented in Table 4 below, NVP-LBH589 demonstrated marked synergy with lapatinib in the Her2+ BT474 and SK-BR-3 cells, and in a less degree, in MDA-MB-453 cells. When tested in combination with the EGFR inhibitor gefitinib that is much less active in the Her2+ cell lines, NVP-LBH589 showed additive effect in BT474 and MDA-MB-453 cells, and some synergistic effect in SK-BR-3 cells. In the Her2- cell line MCF-7, the combinatorial effect of NVP-LBH589 with lapatinib or gefitinib was additive as indicated by the $CI_{50}$ value of 1.

| Combination Index Values at $EC_{50}$ ($CI_{50}$) | | | |
|---|---|---|---|
| **Cell Lines** | **Genotypes** | **NVP-LBH589 + Lapatinib** | **NVP-LBH589 + Gefitinib** |
| BT474 | ER+/Her2+ | 0.4 | 0.9 |
| SK-BR-3 | ER-/Her2+ | 0.38 | 0.6 |
| MDA-MB-453 | ER-/Her2+ | 0.86 | 0.94 |
| MCF-7 | ER+/Her2- | 1 | 1 |

**Example 2:**

Potent antileukemia activity of the combination of the heat shock protein 90 inhibitor NVP-AUY922 and the histone deacetylase inhibitor LBH589 (panobinostat) against human AML and CML cells

NVP-AUY922 is a novel 4,5-diarylsoxazole ATP-binding site heat shock protein 90 (hsp90) inhibitor, which has been shown to inhibit the chaperone function of hsp90 and deplete the levels of hsp90 client proteins. Treatment with AUY922 has been shown to exert potent in vitro anti-tumor activity, as well as in vivo tumor retention and growth inhibitory effects. Present studies demonstrate that AUY922 dose-dependently induced accumulation of human acute myeloid leukemia MV4-11 and Bcr-Abl-expressing K562 cells in G1 and G2/M phases of the cell cycle, with concomitant decline in the percentage of cells in the S phase of the cell cycle (Table 4). In U937 and MV4-11 cells AUY922 dose-dependently (20 to 50 nM) induced apoptosis (40-60% of cells; Figure 1). This was associated with depletion of FLT-3, p-STAT5, AKT, and CDK4 levels, with concomitant induction of hsp70 levels in MV4-11 cells. In K562 cells, treatment with 50-100

nM of AUY922 depleted Bcr-Abl, p-STAT, p-CrkL and AKT levels and induced apoptosis (30-50% of cells). Our previous studies had shown that, by inhibiting histone deacetylase (HDAC) 6, the pan-HDAC inhibitor LBH589 induces acetylation and inhibition of hsp90 chaperone function, resulting in depletion of unmutated or mutant forms of Bcr-Abl, as well as of c-Raf and AKT in CML cells. In the present studies we demonstrate that co-treatment with AUY922 (10 or 20 nM) and LBH589 (5 nM) caused more depletion of FLT-3, p-STAT5 and AKT and synergistically induced apoptosis of MV4-11 cells. Similarly, co-treatment with AUY922 (20 to 100 nM) and LBH589 (50 nM) caused more depletion of Bcr-Abl, p-STAT5, p-CrkL and AKT and synergistically induced apoptosis of K562 cells (by isobologram analyses; Figure 2). Importantly, co-treatment with AUY922 and LBH589 also caused more depletion of the ectopically expressed unmutated Bcr-Abl, mutant Bcr-AblE255K and Bcr-AblT315K, p-STAT5, p-CrkL, p-AKT, c-Raf, as well as synergistically induced apoptosis of BaF3 cells with the unmutated or the mutant forms of Bcr-Abl. Finally co-treatment with AUY922 and LBH589 caused more loss of cell viability than either agent alone in four primary AML samples and five imatinib-refractory, primary CML samples (Table 5). These in vitro studies show that the combination of AUY922 and LBH589 exert synergistic antileukemia activity against human AML and CML cells.

Table 4 MV4-11 and K562 cells were treated with AUY922 for 24 hours. Following this, cells were stained with propidium iodide and cell cycle status was determined by flow cytometry. Values represent the mean ± S.E.M. of cells in G0/G1, S and G2/M of the cell cycle.

| Cells and treatment | % of cells | | |
|---|---|---|---|
| | G0/G1 | S | G2/M |
| MV4-11 | | | |
| Untreated | 63.07±0.52 | 31.05±0.15 | 5.87±0.60 |
| 5 nM AUY922 | 62.23±0.33 | 31.74±0.42 | 6.02±0.74 |
| 10 nM AUY922 | 63.54±1.20 | 30.16±0.39 | 6.30±0.92 |
| 20 nM AUY922 | 85.71 ±2.14 | 8.98±2.41 | 5.30±0.71 |
| 50 nM AUY922 | 79.57±1.15 | 8.47±2.30 | 11.96±1.26 |
| K562 | | | |
| Untreated | 30.88±0.84 | 59.28±0.35 | 9.83±0.53 |
| 20 nM AUY922 | 31.04±3.11 | 60.41±2.01 | 8.54±1.16 |
| 50 nM AUY922 | 61.42±5.30 | 34.14±5.01 | 4.43±2.78 |
| 100 nM AUY922 | 58.30±2.87 | 19.97±0.31 | 21.73±2.67 |
| 250 nM AUY922 | 58.10±4.47 | 20.47±1.54 | 21.38±3.07 |

Table 5 Peripheral blood or bone marrow from 4 CML and 7 AML patients were treated with the indicated doses of AUY922 and/or LBH589 for 48 hours. Then, the percentages of non-viable cells for each drug alone or drug combination were determined by trypan blue uptake in a hemocytometer. Values represent the percentage of non-viable cells from each condition as compared with untreated cells.

| Sample No. | AUY922 and/or LBH589 induces loss of viability of primary AML and CML cells % non-viable cells | | | | | |
|---|---|---|---|---|---|---|
| | Untreated | 20 nmol/L, AUY922 | 50 nmol/L, AUY922 | 50 nmol/L, LBH589 | 20 nmol/L, AUY922 + LBH589 | 50 nmol/L, AUY922 + LBH589 |
| CML#L | 14.7 | 29.1 | 33.6 | 43.3 | 43.1 | 51.7 |
| CML#2 | 6.1 | 9.6 | 13.9 | 21.4 | 23.0 | 29.9 |
| CML#3 | 3.0 | 21.1 | 25.3 | 47.8 | 61.0 | 64.0 |
| CML#4 | 9.0 | 30.5 | 32.8 | 37.6 | 41.7 | 42.4 |
| AML#1 | 3.6 | 38.8 | 45.1 | 80.7 | 81.9 | 83.8 |
| AML#2 | 11.6 | 54.0 | 58.9 | 54.7 | 63.6 | 67.2 |
| AML#3 | 9.5 | 40.1 | 42.7 | 46.3 | 43.0 | 48.0 |
| AML#4 | 10.3 | 43.2 | 50.0 | 53.8 | 58.7 | 60.3 |
| AML#5 | 9.2 | 32.1 | 27.7 | 61.8 | 71.7 | 76.6 |
| AML#6 | 2.4 | 11.9 | 21.4 | 17.8 | 25.6 | 35.9 |

(continued)

| Sample No. | AUY922 and/or LBH589 induces loss of viability of primary AML and CML cells % non-viable cells | | | | | |
|---|---|---|---|---|---|---|
| | Untreated | 20 nmol/L, AUY922 | 50 nmol/L, AUY922 | 50 nmol/L, LBH589 | 20 nmol/L, AUY922 + LBH589 | 50 nmol/L, AUY922 + LBH589 |
| **AML#7CD34enriched** | 10.2 | 70.1 | 70.9 | 56.7 | 80.6 | 92.9 |
| **NormalCD34+cells** | 12.7 | 30.8 | 31.6 | 23.3 | 32.3 | 30.4 |

## Claims

**1.** A combination of:

(a) N-hydroxy-3-[4-[[[2-(2-methyl-1 H-indol-3-yl)-ethyl]-amino]methyl]phenyl]-2E-2-propenamide; and
(b) HSP 90 inhibitor

**2.** The combination of claim 1 wherein the HSP90 inhibitor is); 5-(2,4-Dihydroxy-5-isopropyl-phenyl)-4-(4-morpholin-4-ylmethyl-phenyl)-isoxazole-3-carboxylic acid ethylamide.

**3.** The combination of claim 1 for use in preventing or treating a proliferative disease.

**4.** The combination of Claim 3, wherein the proliferative disease is selected from breast cancer, melanoma, ovarian cancer,lung cancer, pancreatic cancer, myeloma cancer, colorectal cancer, renal cancer, lympohoma, leukemia and colon cancer.

**5.** The combination of claim 4 wherein leukemia is chronic myelogenous leukemia (CML) or acute meyloid leukemia.

**6.** A pharmaceutical composition comprising

(a) N-hydroxy-3-[4-[[[2-(2-methyl-1H-indol-3-yl)-ethyl]-amino]methyl]phenyl]-2E-2-propenamide; and
(b) a HSP90 inhibitor.

**7.** The pharmaceutical composition of claim 5 wherein the HSP90 inhibitor is); 5-(2,4-Dihydroxy-5-isopropyl-phenyl)-4-(4-morpholin-4-ylmethyl-phenyl)-isoxazole-3-carboxylic acid ethylamide.

Europäisches Patentamt
European Patent Office
Office européen des brevets

# EUROPEAN SEARCH REPORT

Application Number
EP 11 15 7791

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GEORGE PRINCE ET AL: "COMBINATION OF THE HISTONE DEACETYLASE INHIBITOR LBH589 AND THE HSP90 INHIBITOR 17-AAG IS HIGHLY ACTIVE AGAINST HUMAN CML-BC CELLS AND AML CELLS WITH ACTIVATING MUTATION OF FLT-3", BLOOD, W.B.SAUNDERS COMPANY, ORLANDO, FL, vol. 105, no. 4, 28 October 2004 (2004-10-28), pages 1768-1776, XP008075090, ISSN: 0006-4971 * abstract * ----- | 1-7 | INV. A61K31/00 A61K31/4045 A61P35/00 A61K31/5377 |
| X | REVILL P ET AL: "Panobinostat: Histone deacetylase (HDAC) inhibitor, apoptosis inducer, oncolytic", DRUGS OF THE FUTURE, BARCELONA, ES, vol. 32, no. 4, 1 January 2007 (2007-01-01), pages 315-322, XP002457526, ISSN: 0377-8282 * abstract * ----- | 1-7 | |
| | | | TECHNICAL FIELDS SEARCHED (IPC) |
| X,P | FISKUS WARREN ET AL: "Potent antileukemia activity of the combination of the heat shock protein 90 inhibitor NVP-AUY922 and the histone deacetylase inhibitor LBH589 (panobinostat) against human AML and CML cells", PROCEEDINGS OF THE ANNUAL MEETING OF THE AMERICAN ASSOCIATION FOR CANCER RESEARCH, AMERICAN ASSOCIATION FOR CANCER RESEARCH, US, vol. 49, 1 April 2008 (2008-04-01), pages 1338-1339, XP001539417, ISSN: 0197-016X * abstract * ----- | 1-7 | A61K A61P |

The present search report has been drawn up for all claims

| Place of search | Date of completion of the search | Examiner |
|---|---|---|
| Munich | 7 July 2011 | Herrera, Suzanne |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4636505 A
- US 4659516 A
- WO 0009495 A
- WO 02092599 A
- US 5093330 A
- WO 9702266 A
- EP 0564409 A
- WO 9903854 A
- EP 0520722 A
- EP 0566226 A
- EP 0787722 A
- EP 0837063 A
- US 5747498 A
- WO 9810767 A
- WO 9730034 A
- WO 9749688 A
- WO 9738983 A
- WO 9630347 A
- WO 9633980 A
- WO 9503283 A
- WO 03013541 A
- US 4100274 A
- US 5843901 A
- WO 0222577 A
- US 5010099 A
- US 6194181 B
- WO 9810121 A
- WO 9825929 A
- WO 9808849 A
- WO 9943653 A
- WO 9822461 A
- WO 0031247 A
- US 5461076 A
- WO 9917804 A1
- WO 9835958 A
- WO 0027820 A
- WO 0059509 A
- WO 9811223 A
- WO 0027819 A
- EP 0769947 A
- WO 0037502 A
- WO 9410202 A


### Non-patent literature cited in the description


- **NANDY et al.** *Acta Oncologica,* 1994, vol. 33 (8), 953-961
- **HELLMAN et al.** Cancer. 1993, vol. 1, 248-275
- **PREWETT et al.** *Cancer Res,* 1999, vol. 59, 5209-5218
- **YUAN et al.** *Proc Natl Acad Sci USA,* 1996, vol. 93, 14765-14770
- **ZHU et al.** *Cancer Res,* 1998, vol. 58, 3209-3214
- **MORDENTI et al.** *Toxicol Pathol,* 1999, vol. 27 (1), 14-21
- **O'REILLY et al.** *Cell,* 1994, vol. 79, 315-328
- **O'REILLY et al.** *Cell,* 1997, vol. 88, 277-285